# EUROPEAN PATENT SPECIFICATION

(11) **EP 4 022 089 B1**
(45) Date of publication and mention of the grant of the patent: **21.08.2024**
(21) Application number: 19839546.9
(22) Date of filing: 18.12.2019
(51) Int. Cl.: C12Q 1/6883, G01N 33/53, G01N 33/68

(54) **METHODS FOR IDENTIFYING A COMPANION ANIMAL AT RISK OF CALCIUM OXALATE STONE FORMATION AND MEDICAL USES FOR REDUCING THE RISK**
VERFAHREN ZUR IDENTIFIZIERUNG EINES HAUSTIERS MIT RISIKO FÜR KALZIUMOXALATSTEINBILDUNG SOWIE MEDIZINISCHE VERWENDUNGEN ZUR VERRINGERUNG DES RISIKOS
PROCÉDÉS D'IDENTIFICATION D'UN ANIMAL DE COMPAGNIE À RISQUE DE FORMATION DE PIERRE D'OXALATE DE CALCIUM ET UTILISATIONS MÉDICALES POUR RÉDUIRE LE RISQUE

(43) Date of publication of application: 06.07.2022
(73) Proprietor: Hill's Pet Nutrition, Inc., Topeka, KS 66603 (US)
(72) Inventor: JEWELL, Dennis, Lawrence, KS 66049 (US); BROCKMAN, Jeffrey, Lawrence, Kansas 66047 (US); EPHRAIM, Eden, Lawrence, Kansas 66049 (US); JACKSON, Matthew, Topeka, Kansas 66604 (US)
(74) Representative: Wichmann, Hendrik
(86) International application number: PCT/US2019/067111
(87) International publication number: WO 2021/126184

(56) References cited:
- WO-A1-2015/072972
- WO-A1-2016/176691
- WO-A1-2018/125539
- JEAN A. HALL ET AL: "Increased dietary long-chain polyunsaturated fatty acids alter serum fatty acid concentrations and lower risk of urine stone formation in cats", PLOS ONE, vol. 12, no. 10, 26 October 2017 (2017-10-26), pages e0187133, XP055678206, DOI: 10.1371/journal.pone.0187133
- ELISA OPPICI ET AL: "Liver peroxisomal alanine:glyoxylate aminotransferase and the effects of mutations associated with Primary Hyperoxaluria Type I: An overview", BIOCHIMICA ET BIOPHYSICA ACTA (BBA) - PROTEINS & PROTEOMICS, vol. 1854, no. 9, September 2015 (2015-09-01), Netherlands, pages 1212 - 1219, XP055463806, ISSN: 1570-9639, DOI: 10.1016/j.bbapap.2014.12.029
- ROMAN N. RODIONOV ET AL: "AGXT2: a promiscuous aminotransferase", TRENDS IN PHARMACOLOGICAL SCIENCES., vol. 35, no. 11, November 2014 (2014-11-01), GB, pages 575 - 582, XP055679796, ISSN: 0165-6147, DOI: 10.1016/j.tips.2014.09.005
- DMITRII TANIANSKII ET AL: "Beta-Aminoisobutyric Acid as a Novel Regulator of Carbohydrate and Lipid Metabolism", NUTRIENTS, vol. 11, no. 3, 28 February 2019 (2019-02-28), CH, pages 524, XP055679757, ISSN: 2072-6643, DOI: 10.3390/nu11030524
- SONGYAN GAO ET AL: "Metabolomics analysis for hydroxy-L-proline-induced calcium oxalate nephrolithiasis in rats based on ultra-high performance liquid chromatography quadrupole time-of-flight mass spectrometry", SCIENTIFIC REPORTS, vol. 6, no. 1, 22 July 2016 (2016-07-22), XP055678850, DOI: 10.1038/srep30142

## Description

### BACKGROUND

Bladder stones are relatively common in cats. Cats that have disease that affects the lower urinary tract, such as struvite or calcium oxalate bladder stones typically have one or more symptoms such as urinating outside the litter box, straining to urinate, frequent urination producing only a small amount of urine at any one time, blood in the urine and genital licking. Results from X-rays, and ultrasound of the abdomen may lead to the discovery of bladder stones. Urinalysis results such as urine pH, the presence of crystals and the absence of infection may lead to the diagnosis of calcium oxalate bladder stones. Bladder stones can be removed by surgery, cystoscopy (in female cats) or lithotripsy. A sample of the stones can be analyzed to confirm the diagnosis.

Historically, bladder stones in cats were made of struvite, which is magnesium ammonium phosphate. To prevent struvite bladder stones, many feline diets are formulated to make cats produce more acidic urine, which dissolves struvite and/or prevents struvite stone formation. Such diets result in urinary acidification which is very effective to prevent struvite stone formation. An undesirable consequence of such diets, however, is that they also create high blood calcium levels that put cats at increased risk for calcium oxalate stones. That is, many of the feline diets formulated to prevent struvite stone formation also increase conditions that promote calcium oxalate stones.

Calcium oxalate stones are a significant cause of death and discomfort in cats. While the incidence of calcium oxalate bladder stones can be reduced by feeding that cat a non-acidifying diet, such a diet increases the risk of struvite bladder stones. Diets with a high-water content and/or supplemented with potassium citrate, may result in a urine with a pH over 6.5, a specific gravity of about 1.020, and an absence of crystals, may reduce risk of calcium oxalate stone formation. Such diets, however, are often insufficient to significantly reduce risk of calcium oxalate stones.

Strategies to reduce calcium oxalate stone formation include those that address any underlying condition that leads to high blood calcium levels. Calcium oxalate stones form from calcium oxalate crystals. Oxalate is the product of oxidation of glyoxylate.

In addition to be being a substrate to form oxalate, glyoxylate may alternatively be catalyzed to glycine by alanine-glyoxylate aminotransferase 2 (AGTX2) using L-alanine as the amino donor. AGTX2, which is encoded by the AGXT2 gene, is a class III pyridoxal-phosphate-dependent mitochondrial aminotransferase, which is considered a promiscuous aminotransferase. AGTX2 is an important regulator of methylarginines and is involved in the control of blood pressure in the kidney. By metabolizing glyoxylate into products other than oxalate, enzymes such as AGXT, AGXT2 and others reduce the level of glyoxylate that would otherwise be available as a substrate for oxalate production, and accordingly activity by such enzymes reduce oxalate levels.

Polymorphisms in the AGXT2 gene affect methylarginine and beta-amino isobutyrate metabolism influencing concentration of dimethylarginines and beta-amino isobutyrate. In addition, AGXT2 polymorphisms are associated with carotid atherosclerosis.

Symmetric dimethyl arginine (SDMA) is a substrate of AGXT2. SDMA has been shown to be associated (increased) with changes in renal function in the cat (prior to a decline in renal function) (Hall et al, 2014, Comparison of Serum Concentrations of Symmetric Dimethylarginine and Creatinine as Kidney Function Biomarkers in Cats with Chronic Kidney Disease J Vet Intern Med;28:1676-1683; Hall et al., 2014, Comparison of serum concentrations of symmetric dimethylarginine and creatinine as kidney function biomarkers in healthy geriatric cats fed reduced protein foods enriched with fish oil, L-carnitine, and medium-chain triglycerides The Veterinary Journal 202 588-596) as well as showing a relationship between SDMA and stone formation in the cat (Hall et al, 2017 Serum concentrations of symmetric dimethylarginine and creatinine in cats with kidney stones PLoS ONE 12 (4): e0174854).

Some researchers, noting that SDMA and ADMA are substrates of AGXT2, have shown an association with AGXT2 polymorphisms and their concentration. For example, Kittle et al. (2014) Alanine-glyoxylate aminotransferase 2 (AGXT2) Polymorphisms Have Considerable Impact on Methylarginine and β-amino isobutyrate Metabolism in Healthy Volunteers, PLoS ONE 9(2):e88544 showed that specific polymorphisms influenced concentration of dimethylarginines and beta-amino isobutyrate and were associated with beta-amino isobutyrate concentrations as well as SDMA concentrations.

A significant positive interaction with the dimethylarginines and beta-amino isobutyrate has been noted. The association with methylarginines and AGXT2 polymorphisms was also shown by Zhou et al (2014) Association of the AGXT2 V140I Polymorphism with risk for coronary Heart Disease in a Chinese Population, J Atheroscler Thromb. 21(10):1022-30. In particular increased ADMA was shown to be influenced by AGXT2 genotypes in a population of smokers and those with diabetes mellitus.

A urine calcium oxalate titrimetric test (COTT) is a method that is useful to assess risk of calcium oxalate stone formation (Hall et al, 2017 Increased dietary long-chain polyunsaturated fatty acids alter serum fatty acid concentrations and lower risk of urine stone formation in cats. PLoS ONE 12(10):e88544). The concentration of ionized calcium and the amount of oxalate that is added to initiate crystallization per liter in a urine sample is calculated as a ratio [Ca⁺²]/(added Ox⁻²). An increasing index value denotes samples at greater risk of calcium oxalate crystallization, whereas decreasing index values denotes those with less risk. Calcium oxalate titration test (COTT) results can be improved by dietary intervention to lower the stone formation risk for calcium oxalate stones in cats

Single nucleotide polymorphisms (SNPs) are a common type of genetic variation. SNPs are single base pair mutations at a specific locus. That is, a SNP is a difference in a single nucleotide in a DNA sequence that occurs at a specific position in a genome. Typically, for a SNP at a specific position, there are two possible nucleotide variations, which are referred to as alleles for that position. Within a population, the nucleotide variation that most commonly appears at a specific base position in a genome is referred to as the major allele; the nucleotide variation that is less common at that specific base position is referred to as the minor allele. Felines, like most multicellular organisms have two sets of chromosomes. Thus, each cat has two copies of each gene or locus and therefore two copies of each SNP. Accordingly, for each SNP in the cat's genome, the cat may have two copies of the major allele, or one copy of the minor allele and one copy of the minor allele, or two copies of the minor allele.

SNPs can act as biological markers. Some SNPs have been found helpful in predicting drug responses and risk of developing particular diseases. SNP genotyping refers to detection of SNPs within the genome. There are numerous methods for detecting SNPs and performing SNP genotyping.

There is a need to develop improved methods to identify cat having increased likelihood or risk of developing calcium oxalate stones. There is a need to develop methods to determine if a cat has a relatively high, intermediate or low likelihood or risk of developing calcium oxalate stones. There is a need for improved compositions and methods that reduce the risk of developing calcium oxalate bladder stones, including compositions and methods that reduce the risk of developing calcium oxalate bladder stones in at least a subpopulation of cats having genetic markers or phenotypic traits. There is a need for improved compositions and methods that prevent calcium oxalate stone formation in at least a subpopulation of cats having genetic markers or phenotypic traits. There is a need to develop methods to identify cats that will benefit from treatment to reduce the likelihood or risk of developing calcium oxalate stones. There is a need for kits, reagents, other articles, and compositions useful in such methods.

WO2016/176691 disclosed that elevated BAIB serum concentration was indicative of kidney stones.

### BRIEF SUMMARY

Methods that comprise analyzing a biological sample obtained from the feline subject for the presence of one or two copies of minor allele A of SNP A1_212891692 are provided. A feline subject having one or two copies of minor allele A of SNP A1_212891692 indicates that the feline subject has an increased likelihood or risk of calcium oxalate stone formation. In some embodiments, the sample is analyzed by performing DNA sequencing, restriction enzyme digest, polymerase chain reaction (PCR), hybridization, real-time PCR, reverse transcriptase PCR, or ligase chain reaction. In some embodiments, the sample is a genomic DNA sample. In some embodiments, the sample is obtained from blood, saliva, follicle root, nasal swab or oral swab of the feline subject, preferably saliva. In some embodiments, the sample is analyzed by performing at least one nucleic acid analysis technique selected from: analysis using a whole genome SNP chip, single-stranded conformational polymorphism (SSCP) assay, restriction fragment length polymorphism (RFLP), automated fluorescent sequencing; clamped denaturing gel electrophoresis (CDGE); denaturing gradient gel electrophoresis (DGGE), mobility shift analysis, restriction enzyme analysis, heteroduplex analysis, chemical mismatch cleavage (CMC), RNase protection assays, use of polypeptides that recognize nucleotide mismatches, allele-specific PCR, sequence analysis, and SNP genotyping. In some embodiments, the sample is analyzed by performing at least one nucleic acid analysis technique selected from: hybridization-based methods, enzyme-based methods, post-amplification methods based on physical properties of DNA, and sequencing methods.

Methods that comprise analyzing a biological sample obtained from the feline subject to determine the feline subject's beta-amino isobutyrate (BAIB) concentration are disclosed but not claimed. The feline subject's BAIB concentration may be compared to a positive reference BAIB concentration value or a measured value from a positive control. The positive reference BAIB concentration value that is representative of BAIB concentration of a cat that has an increased likelihood or risk of calcium oxalate stone formation. The positive control sample contains BAIB at a concentration representative of the BAIB concentration of a cat that has an increased likelihood or risk of calcium oxalate stone formation. A feline subject with BAIB concentration being equal to or greater than the positive reference BAIB concentration value or the measured BAIB concentration of the positive control indicates that the feline subject has an increased likelihood or risk of calcium oxalate stone formation.

Methods that comprise analyzing a biological sample obtained from the feline subject to determine the feline subject's 2-oxoarginine concentration are provided. The feline subject's 2-oxoarginine concentration may be compared to a positive reference 2-oxoarginine concentration value or a measured value from a positive control. The positive reference 2-oxoarginine concentration value that is representative of 2-oxoarginine concentration of a cat that has an increased likelihood or risk of calcium oxalate stone formation. The positive control sample contains 2-oxoarginine at a concentration representative of the 2-oxoarginine concentration of a cat that has an increased likelihood or risk of calcium oxalate stone formation. A feline subject with 2-oxoarginine concentration being equal to or less than the positive reference 2-oxoarginine concentration value or the measured 2-oxoarginine concentration of the positive control indicates that the feline subject has an increased likelihood or risk of calcium oxalate stone formation.

Methods of reducing risk of calcium oxalate stone formation in a feline subject are disclosed but not claimed. The methods comprise the steps of identifying the feline subject as being a feline subject that has an increased likelihood or risk of calcium oxalate stone formation; and feeding the feline subject a low arginine diet. The feline subject is identified as being a feline subject that has an increased likelihood or risk of calcium oxalate stone formation by detecting the presence of one or two copies of minor allele A of SNP A1_212891692 and/or detecting the feline subject's BAIB level and determining it indicates that feline subject has an increased likelihood or risk of calcium oxalate stone formation. In some embodiments, the feline subject is fed a low arginine diet that contains 1.84% or less of arginine as a percentage of nutritional intake based on the dry matter daily intake.

Feline food compositions that comprise 1.84% or less of arginine based on dry weight are disclosed but not claimed.

### DETAILED DESCRIPTION

The following description of the preferred embodiment(s) is merely exemplary in nature and is in no way intended to limit the invention, its application, or uses.

As used herein and in the appended claims, the singular forms "a", "an", and "the" include plural reference unless the context clearly dictates otherwise.

The term "cat" includes those cats which are companion animals known as domestic cats or house cats, or *Felis domesticus.* The term cat is synonymous with the term feline.

As described herein, increased likelihood or risk of developing calcium oxalate stones refers to having a greater than average chance that an individual cat will develop calcium oxalate stones compared to that of a population of cats. The incidence of calcium oxalate stones in cats that have the AA genotype is greater than the incidence of calcium oxalate stones in cats that have the GG. That is, in a population of cats with the AA genotype, a larger fraction develops calcium oxalate stones compared to the fraction of cats with the GG genotype that develop calcium oxalate stones. As set forth below, in a study of described herein, cats with the GG genotype had the lowest incidence of stone formation (8.5%), cats with the AG genotype had an intermediate level of incidence (12.6%) and cats with the AA genotype had the highest level of incidence (23.3%). Accordingly, cats with the AA and AG genotypes have an increased likelihood or risk of developing calcium oxalate stones.

Methods for identifying a feline subject with an increased likelihood or risk of calcium oxalate stone formation and for treating such feline subjects in order to reduce risk of calcium oxalate stone formation are disclosed but not claimed. The treatment methods comprise the step of feeding the feline subject a low arginine diet, which is in some embodiments also high in fiber. In some embodiments, the methods for identifying a feline with an increased likelihood or risk of calcium oxalate stone formation and for treating such feline subjects in order to reduce risk of calcium oxalate stone formation comprise analyzing a sample from the feline subject to determine if the feline subject has an AA or AG genotype for SNP A1_212891692 (felCat8). In some embodiments, the methods for identifying a feline subject that will benefit from such treatment to reduce the likelihood of developing calcium oxalate bladder stones comprise analyzing a sample from the feline subject to determine if the level of circulating beta-amino isobutyrate (BAIB) indicates that the feline subject that will benefit from the treatment.

Methods for treating a feline subject with an increased likelihood or risk of calcium oxalate stone formation to reduce the likelihood of developing calcium oxalate bladder stones are disclosed but not claimed. The treatment to reduce risk of calcium oxalate stone formation that comprises feeding the feline subject a low arginine diet, which is some embodiments has high fiber content. In some embodiments, the methods comprise the steps of identifying a feline subject as an increased likelihood or risk of calcium oxalate stone formation to reduce the likelihood of developing calcium oxalate bladder stones and then feeding the feline subject a low arginine diet, which is some embodiments has high fiber content. A feline subject may be identified as having an increased likelihood or risk of calcium oxalate stone formation by determining if the feline subject has an AA or AG genotype for SNP A1_212891692 (felCat8) and/or determining if the level of circulating BAIB indicates that the feline subject that will benefit from the treatment.

Compositions that can serve as a daily diet having low arginine content are disclosed but not claimed. The compositions useful in the methods may be a cat food composition.

As used herein, low arginine diet refers to a daily diet having a low level of arginine as expressed as a percent total daily nutritional intake based on percent of total dry weight (not calories). A low level of arginine corresponds to 1.84% or less of arginine as a percent of total daily nutritional intake based on percent of total dry weight. In some embodiments, low arginine diet refers to 1.04-1.84% arginine as a percent of total daily nutritional intake based on percent of total dry weight. In some embodiments, low arginine diet refers to a daily diet having 1.21% or less arginine as expressed as a percent total daily nutritional intake based on percent of total dry weight. In some embodiments, low arginine diet refers to a daily diet having 1.11% or less arginine as expressed as a percent total daily nutritional intake based on percent of total dry weight. In some embodiments, the compositions include food compositions contain less than 1.04% arginine based on the total weight of the composition on a dry matter basis. Table 14 below is an excerpt from Table 10 that appears in the reference text "Nutrient Requirements of Domestic Animals: Nutrient Requirements of cats, Revised Edition, National Academy Press Washington. DC. 1986. page 63-67" which lists the percentage of particular amino acids in various protein sources used in cat food compositions. The excerpted portion in Table 14 shows the % arginine. Those skilled in the art could readily formulate a suitable cat food to provide the daily nutritional requirements for a cat as disclosed in Official Publication of the Association of American Feed Control Officials, Inc. (AAFCO), Atlanta, Ga., (2012). Using the table in "Nutrient Requirements of Domestic Animals: Nutrient Requirements of cats, Revised Edition. National Academy Press Washington. DC. 1986. page 63-67" which is designated Table 10 in the reference and excerpted in relevant part in Table 14 herein, those skilled in the art could readily formulate a suitable cat food to provide the daily nutritional requirements while restricting the level of arginine to 1.84% or less of the total daily nutritional intake based on percent of total dry weight, and in some embodiments arginine at a level of arginine between 1.04-1.84% of the total daily nutritional intake based on percent of total dry weight, and in some embodiments arginine at a level of 1.21% or less of the total daily nutritional intake based on percent of total dry weight, and in some embodiments arginine at a level of 1.11% or less of the total daily nutritional intake based on percent of total dry weight

A "food," "food composition," "pet food composition" or "cat food composition" can, in some embodiments, be a nutritionally complete diet for cat to which it is fed.

As used herein, an "ingredient" refers to any component of a composition.

The term "nutrient" refers to a substance that provides nourishment. In some cases, an ingredient may comprise more than one "nutrient," for example, a composition may comprise corn comprising important nutrients including both protein and carbohydrate.

Food compositions can be provided to in the form of cat food. A variety of commonly known types of cat foods are available to cat owners. The selection of cat food includes but is not limited to wet cat food, semi-moist cat food, dry cat food and cat treats. Wet cat food generally has a moisture content greater than about 65%. Semi-moist cat food typically has a moisture content between about 20% and about 65% and may include humectants, potassium sorbate, and other ingredients to prevent microbial growth (bacteria and mold). Dry cat food such as but not limited to food kibbles generally has a moisture content below about 15%. Pet treats typically may be semi-moist, chewable treats; dry treats in any number of forms, or baked, extruded or stamped treats; confection treats; or other kinds of treats as is known to one skilled in the art.

As used herein, the term "kibble" or "food kibble" refers to a particulate pellet like component of cat feeds. In some embodiments, a food kibble has a moisture, or water, content of less than 15% by weight. Food kibbles may range in texture from hard to soft. Food kibbles may range in internal structure from expanded to dense. Food kibbles may be formed by an extrusion process or a baking process. In non-limiting examples, a food kibble may have a uniform internal structure or a varied internal structure. For example, a food kibble may include a core and a coating to form a coated kibble. It should be understood that when the term "kibble" or "food kibble" is used, it can refer to an uncoated kibble or a coated kibble.

As used herein, the term "extrude" or "extrusion" refers to the process of sending preconditioned and/or prepared ingredient mixtures through an extruder. In some embodiments of extrusion, food kibbles are formed by an extrusion processes wherein a kibble dough, including a mixture of wet and dry ingredients, can be extruded under heat and pressure to form the food kibble. Any type of extruder can be used, examples of which include but are not limited to single screw extruders and twin-screw extruders. The list of sources, ingredients, and components as described hereinafter are listed such that combinations and mixtures thereof are also contemplated and within the scope herein.

As contemplated herein, compositions are meant to encompass, but not be limited to, nutritionally-complete and balanced cat food compositions. A "nutritionally complete diet" is a diet that includes sufficient nutrients for maintenance of normal health of a healthy cat on the diet. Nutritionally complete and balanced cat food compositions are familiar to one of skill in the art. For example, substances such as nutrients and ingredients suitable for nutritionally complete and balanced animal feed compositions, and recommended amounts thereof, may be found for example, in the Official Publication of the Association of American Feed Control Officials. Inc. (AAFCO), Atlanta, Ga., (2012).

### Genetic factors indicating responsiveness to treatment for reducing risk

Genetic factors have been identified as being associated with an increased likelihood or risk of calcium oxalate stone formation. Through a genome wide study of a feline colony, a single nucleotide polymorphism (SNP A1_212891692, felCat8; corresponding to SNP A1_212069607, felCat5), which is downstream from the AGXT2 gene, has been identified and associated with an increased likelihood or risk of calcium oxalate stone formation and whether or not a treatment comprising a low arginine diet can be effective to reduce the risk. In particular, the presence of two copies of the minor allele (the AA genotype) is associated with a high likelihood or risk of calcium oxalate stone formation; the presence of one copy of the minor allele and one copy of the major allele (the AG genotype) is associated with an intermediate likelihood or risk of calcium oxalate stone formation. Cats with the AA and AG genotypes are particularly responsive to treatments described herein to reduce risk of calcium oxalate stone formation in cats.

A genome wide association analysis was performed which showed that there was a relationship between the AGXT2 (alanine-glyoxylate aminotransferase 2) gene and circulating concentrations of 2-oxoarginine, which is the product of the transamination of arginine. The AA and AG genotypes had increased concentration of beta-amino isobutyrate (BAIB) when compared to the GG genotype. The protein encoded by AGXT2 is a class III pyridoxal-phosphate-dependent mitochondrial aminotransferase. It is known as a catalyst for the conversion of glyoxylate to glycine using L-alanine as the amino donor. The polymorphisms in this gene are also associated with BAIB concentration. AGXT2 has one of the dimethyl arginines (ADMA) as a substrate and BAIB as a product. The AGXT2 polymorphisms are related not only to BAIB concentration but also stone formation.

The GG genotype has the lowest incidence of stone formation (8.5%), AG intermediate (12.6%) and AA the highest (23.3%). These higher incidences of stone formation were associated with increasing BAIB concentrations. In relative units, the concentration of BAIB in the AA genotype was 6.8, in cats with AG it was 3.0 and in cats with GG the BAIB concentration was 1.7. Cats that developed a stone within each genotype had a higher concentration of BAIB than the non-stone forming cats in that genotype. Using each genotype as a unit there was a 38% average increase in BAIB concentration in the stone forming cats. In summary, the genotype with the highest BAIB experienced the highest level of stone formation and within each genotype the stone forming cats had a higher concentration of BAIB.

Cats with the AA or AG genotype of the AGXT2 polymorphism display a significant reduction of BAIB, a biomarker of calcium oxalate stone risk, through reduction of the dietary amino acid arginine. Raw data is show in in Tables 17-20 below.

A relationship between AGXT2 polymorphism and circulating BAIB levels has also been observed.

Cats with the AA and AG genotypes are responsive to a treatment to reduce risk of calcium oxalate stone formation that comprises administering a low arginine diet. Accordingly, determining the particular genotype of in the SNP A1_212891692 (felCat8), which is downstream of the feline AGTX2 gene, in a sample from a feline subject provides an accurate basis for determine whether or not the feline subject has a higher likelihood or risk of developing calcium oxalate stones and therefore will benefit from a low arginine diet.

Feline genetic polymorphism at SNP A1_212891692 can be used to identify a feline subject as either being a cat higher likelihood or risk of developing calcium oxalate stones. A cat that has the AA or AG genotype of SNP A1_212891692 will benefit from a low arginine diet. The AA genotype, (also referred to as two-minor allele genotype or the homozygous minor allele genotype) refers to a cat having the minor allele A present in both copies of SNP A1_212891692. The AG genotype, (also referred to as minor-major allele genotype or the heterozygous genotype) refers to a cat having one copy of the minor allele A and one copy of the major allele G present in both copies of SNP A1_212891692.

The genotypic differences translate to detectable phenotypic differences. Cats that have the AA and AG genotype have detectable differences circulating BAIB levels compared to levels in cats that have the GG genotype. Cats that have the AA genotype have higher levels of BAIB compared to the levels of BAIB from biological samples from cats that have the GG genotype. Cats with the AG genotype have BAIB levels less than those of the AA genotype and greater than those with the GG genotype.

Accordingly, in addition to determining genotype in order to identify a cat as having a higher likelihood or risk of developing calcium oxalate stones, the phenotypic difference, i.e. BAIB level, can also be used to identify whether a feline subject has a higher likelihood or risk of developing calcium oxalate stones.

Genotypic analysis and phenotypic analysis can be used individually, or in combination to provide confirmation and thus a higher level of accuracy in identifying a feline subject as having a higher likelihood or risk of developing calcium oxalate stones and in methods to reduce likelihood or risk of developing calcium oxalate stones in such cats by feeding a low arginine diet. In some embodiments samples from a cat as analyzed for genotype. To identify a feline subject as a cat with a higher likelihood or risk of developing calcium oxalate stones, one or more of the following analyses may be performed: genotypic analysis using a sample from the feline subject may be undertaken to determine that a feline subject has the AA or AG genotype and/or the phenotypic analysis using a sample from the feline subject may be undertaken to identify BAIB levels that indicate a feline subject is a cat with higher likelihood or risk of developing calcium oxalate stones;. In some embodiments, only genotypic analysis is performed. In some embodiments, a combination of genotypic analysis and analysis of BAIB levels is performed.

The treatment used to reduce the likelihood or risk of developing calcium oxalate stones in a feline subject identified as having a higher likelihood or risk of developing calcium oxalate stones comprises feeding the feline subject a low arginine diet. Methods of treating cats to reduce the likelihood of developing calcium oxalate stones that comprise identifying a feline subject as a cat having a higher likelihood or risk of developing calcium oxalate stones cat that and feeding the cat a low arginine diet are disclosed but not claimed. In some embodiments, the low arginine diet is also a high fiber diet. In some embodiments, treatment methods comprise the steps of: 1) either identifying a cat as a cat having higher likelihood or risk of developing calcium oxalate stones by performing a) a genotypic analysis of SNP A1_212891692 and/or b) a phenotypic analysis to identify BAIB levels indicative of an increased likelihood of developing calcium oxalate stones; and 2) feeding the cat a low arginine diet.

Kits, reagents, other articles, and compositions useful in methods for identifying a feline subject as either being a cat with a higher likelihood or risk of developing calcium oxalate stones are disclosed but not claimed. The kits, reagents, other articles, and compositions may be useful in methods that evaluate genotype and/or methods that evaluate BAIB levels. Kits, reagents, other articles, and compositions useful in methods that evaluate BAIB levels may comprise reagents useful in assays to measure such BAIB levels and may also comprise positive control samples and negative control samples.

Kits, reagents, other articles, and compositions useful methods for treating feline subjects to prevent, reduce the likelihood, delay the onset, and/or reduce the severity of calcium oxalate stone formation are disclosed but not claimed. The feline subjects may be identified as having an increased likelihood of developing calcium oxalate stones or as being likely to benefit from a treatment that reduces risk of calcium oxalate stone formation by methods provided herein. The methods of treating feline subjects to prevent, reduce the likelihood, delay the onset, and/or reduce the severity of calcium oxalate stone formation may comprises feeding the feline subjects a low arginine diet.

There is an association between the genotype of a cat at SNP A1_212891692 (felCat8) on chromosome 1 downstream from the downstream of the AGXT2 gene, beta-amino isobutyrate (BAIB) concentration and the incidence of calcium oxalate stone formation. As shown in Table 1, cats having two copies of the minor allele (the AA genotype) had the highest incidence of calcium oxalate stone formation. Cats having two copies of the major allele (the GG genotype) had the lowest incidence of calcium oxalate stone formation. The incidence of calcium oxalate stone formation in cats having one copy of the major allele and one copy of the minor allele (the AG genotype) was between the two homozygous genotypes. These polymorphisms in the AGTX2 gene are also associated with BAIB concentration. Cats with the AA genotype had increased BAIB concentration when compared to the BAIB concentration in cats with AG genotypes. Cats with the GG genotype had the lowest BAIB concentration compared the BAIB concentration of cats with the AG and AA genotypes. Table 1 shows relative concentration of BAIB.

**TABLE 1**

| Genotype | AA | AG | GG |
|---|---|---|---|
| Number of cats | N=30 | N=167 | N=248 |
| Incidence of calcium oxalate stone formation | 23.3% | 12.6% | 8.5% |
| Concentration of BAIB in Relative Units | 6.8 | 3.0 | 1.7 |

The association between incidence of calcium oxalate stone formation and BAIB concentration was analyzed within each genotype. Cats that developed a calcium oxalate stones within each genotype had a higher concentration of BAIB than the non-stone forming cats in that genotype (Table 2). Using each genotype as a unit, there was a 38% average increase in BAIB concentration in the stone forming cats.

**TABLE 2**

| Genotype | AA Genotype | | AG Genotype | | GG Genotype | |
|---|---|---|---|---|---|---|
| Stone | No | Yes | No | Yes | No | Yes |
| Number of cats | N=23 | N=7 | N=146 | N=21 | N=227 | N=21 |
| BAIB Mean | 6.74 | 6.96 | 2.64 | 5.20 | 1.72 | 1.95 |

The genotype with the highest BAIB (the AA genotype) experienced the highest level of stone formation (Table 1) and within each genotype the stone forming cats had a higher concentration of BAIB (Table 2). Because of the higher incidence of calcium oxalate stone formation, there is a significant decrease in health of the cats carrying the A allele in the AGXT2 gene. The higher incidence of calcium oxalate stone formation is related to the increased circulating concentration of BAIB. Methods disclosed but not claimed herein reduces and thereby improves BAIB levels in felines with the AA and AG genotypes. The treatment is directed to reduce the incidence of calcium oxalate stones associated with this polymorphism.

Individual felines having a particular genotype of in the SNP A1_212891692 (felCat8), which is downstream of the feline AGTX2 gene, provides an accurate basis for assessing likelihood or predicting relative risk of calcium oxalate stone formation in a feline. Feline genetic polymorphism as SNP A1_212891692 (felCat8) can be used to identify cats that have an increased risk of developing calcium oxalate stones. The homozygous minor allele genotype AA may also be referred to as two-minor allele genotype or AA genotype. The heterozygous major-minor allele genotype AG may also be referred to as major-minor allele genotype or AG genotype. The homozygous major allele genotype GG may also be referred to as two-major allele genotype or GG genotype.

There is an association between genotype and likelihood of developing calcium oxalate stones. Cats with the AA or AG genotype have an increased risk of developing calcium oxalate stones compared to cats with the GG genotype. Between the two higher risk genotypes, cats with the AA genotype have an increased risk of developing calcium oxalate stones compared to cats with the AG genotype. Thus, the order of likelihood or risk of developing calcium oxalate stones is that cats with the AA genotype have the highest risk or likelihood and may also be referred to as being high risk or high likelihood. Cats with the GG genotype have the lowest risk or likelihood and may also be referred to as being low risk or low likelihood. Cats with the AG genotype have the intermediate or medium risk or likelihood.

Accordingly, a feline subject may be identified as having an increased risk of developing calcium oxalate stones by analyzing a sample from the feline subject and identifying one or two copies of the minor allele A. Genotypes AG and AA have increased risk of developing calcium oxalate stones. A feline subject identified as having two copies of the minor allele A, i.e. the AA genotype may be referred to as having a high risk or high likelihood of developing calcium oxalate stones. A feline subject identified as having one copies of the minor allele A, i.e. the AG genotype may be referred to as having a moderate, medium or intermediate risk or likelihood of developing calcium oxalate stones. A feline subject identified as having zero copies of the minor allele A, i.e. the GG genotype may be referred to as having a low risk or low likelihood of developing calcium oxalate stones.

There is an association between genotype and BAIB levels. Cats that have the AA genotype have highest BAIB concentrations. Cats that have the GG genotype have lowest BAIB concentrations. Cats with AG genotype typically have higher BAIB concentrations than cats that have the GG genotype, and lower BAIB concentrations that cats with the AA genotype.

Cats with the AA or AG genotype of the AGXT2 polymorphism experience a significant benefit by the reduction of the dietary amino acid arginine. A reduction of the dietary amino acid arginine results in lower BAIB levels and a reduced incidence of calcium oxalate stone formation in cats with the AA or AG genotype of the AGXT2 polymorphism. Cats fed a low arginine diet have a reduced risk or reduced likelihood of developing calcium oxalate stones.

Therefore, methods are provided for identifying a feline subject as having an increased risk or likelihood of developing calcium oxalate stones by detection of the AA or AG genotype. Methods are provided for identifying a feline subject as having high risk or likelihood, intermediate risk or likelihood, or low risk or likelihood of developing calcium oxalate stones by detection of the AA, AG or GG genotype, respectively. Methods are provided for identifying a feline subject as a feline subject likely to benefit from the low arginine diet treatment to reduce the likelihood of developing calcium oxalate stones by detection of the AA or AG genotype. Conversely, methods for identifying a feline subject as not a cat likely to benefit from a low arginine diet treatment to reduce the likelihood of developing calcium oxalate stones may comprise detection of the GG genotype. Methods are also disclosed but not claimed for reducing the likelihood of developing calcium oxalate stones by a) identifying a feline subject as being a feline subject likely to benefit from such treatment, wherein the feline subject is identified as having the AA or AG genotype and b) feeding the feline subject a diet that has reduced levels of dietary amino acid arginine.

SNP A1_212891692 in reference genome felCat8 (formerly referred to as SNP A1_212069607 in reference genome felCat5

SNP A1_212891692 refers to the SNP of the feline chromosome A1 sequence downstream of the AGXT2 gene listed in the feline reference genome felCat8. In that database, SNP A1_212891692 refers to Chromosome A1 Base Position 212891692. SNP A1_212891692 is located in a sequence downstream of the AGXT2 gene.

FelCat8 is an improved reference genome for cat that was re-assembled using new DNA sequence for cat. FelCat5 is a previous reference genome for cat. SNP A1_212891692 in reference genome felCat8 corresponds to the SNP formerly referred to as SNP A1_212069607 in reference genome felCat5. The sequence in felCat5 that includes the SNP is updated in felCat8. The felCat8 sequence is more complete.

SNP A1_212891692 refers to the SNP located on Chromosome A1 Base Position 212891692 in felCat8. (SNP A1_212891692 in felCat8 is SNP A1_212069607 in felCat5, which refers to felCat5 Chromosome A1 Base Position 212069607.)

SEQ ID NO:1 is the sequence in felCat8 that includes SNP A1_212891692. SEQ ID NO:1 is chrA1:212891592-212891792 in felCat8.

>felCat8_dna range=chrA1:212891592-212891792 5'pad=0 3'pad=38 strand=+ repeatMasking=none

SEQ ID NO:1:

ATATGTTAGT ATCTCTACAT GTGGGAGAAC CAGATGTCAG 40
GTTCATGTAT GATACAGCAG GAAGAACACA GCACGGCCTT 80
TGAAGTATTC CTGTTTATAG [A/G]AATAATTCT TTCATATGCA 120
GGTACGTGTG TGTGTGTGTG TGTGTGTGTG TGTGTGTGTG 160
TGTGTATTTT ATAAAGGTAG CTACTCCTTA TTCATAGATA T 201

In SEQ ID NO:1, the SNP is at position 101 (of 201), which corresponds to Chromosome A1 at position 212891692. SEQ ID NO:1 contains 100 nucleotides 5' of the SNP and 100 nucleotides 3' of the SNP. The SNP at nucleotide 101 of SEQ ID NO:1, (position 212891692 of Chromosome A1 felCat8) is shown with the minor allele A and the major allele G as alternative nucleotides.

As used herein, when referencing the SNP that is the subject of the polymorphism and genotyping referred to herein, SNP A1_212891692 is intended to refer to the same SNP presented in felCat8 and felCat5 (SNP A1_212069607). In felCat8 the SNP is located at chromosome 1, position 212891692; in felCat5 the SNP is located at chromosome 1, position 212069607.

The presence one A allele and one G allele (the AG genotype) or the presence two A alleles (the AA genotype) based upon SNP detection in an individual feline's genome can be used to identify a feline subject as having a higher likelihood or risk of developing calcium oxalate stones.

A feline subject is identified as being a cat having a higher likelihood or risk of developing calcium oxalate stones by genotyping results that indicate the feline subject has the AA or AG genotype. To genotype a feline subject, in some embodiments, a sample from the feline subject can be interrogated for the presence of both the G allele and the A allele. Detection of the presence of the G allele and the absence of the A allele indicates the GG genotype. Detection of the presence of the G allele and the A allele indicates the AG genotype. Detection of the absence of the G allele and the presence of the A allele indicates the AA genotype. In some embodiments, a sample from the feline subject can be interrogated for the presence of the G allele only; thereby only indirectly determining whether the A allele is present. Detection of the absence of the G allele indicates the AA genotype.

A feline subject is identified as being a cat having a higher likelihood or risk of developing calcium oxalate stones by interrogating a biological sample obtained from the feline subject for the presence of SNP A1_212891692 major allele G and SNP A1_212891692 minor allele A, thereby providing for determining the feline subject has the GG genotype, the AG genotype or the AA genotype. Alternatively, a biological sample obtained from the feline subject may be interrogated for the presence of two copies of a SNP A1_212891692 major allele G only. In such instances, detecting zero copies of a SNP A1_212891692 major allele G is an indirect detection of two AAs and thus the AA genotype. When only interrogating for major allele G, the detection of presence of a major allele G indicates that the feline subject has either the GG genotype or the AG genotype; thus it is only useful in the context of the present invention as a way to identity subjects with the AA genotype, which is the highest risk group.

One skilled in the art, using the coordinates disclosed in the sequence from the reference genome can determine the genotype of a subject. Methods of determining the genotype with respect to single nucleic acid polymorphism SNP A1_212891692 are provided. Those skilled in the art could, using the information provided herein, determine the genotype of an individual feline. In some embodiments, the sample used to perform the genotype analysis is a genomic DNA sample. In some embodiments, the sample is obtained from blood, saliva, follicle root, nasal swab or oral swab of the feline subject. In some embodiments, the biological sample is a genomic DNA sample obtained from the feline subject using the commercially available kit such as PERFORMAgene PG-100 Oral sample collection it (DNA Genotek, OraSure Technologies, Inc., Bethlehem, PA). The preferred method for determining the presence or absence of specific alleles as a single SNP is a PCR based assay using an allele specific fluorescent probe to determine which variant(s) are present in the sample.

In some embodiments, genotype on an individual cat for SNP A1-212069607 may be determined using methods that include at least one nucleic acid analysis technique selected from: DNA sequencing, restriction enzyme digest, polymerase chain reaction (PCR), hybridization, real-time PCR, reverse transcriptase PCR, or ligase chain reaction.

In some embodiments, genotype on an individual cat for SNP A1-212069607 may be determined by performing at least one nucleic acid analysis technique selected from the group consisting of: analysis using a whole genome SNP chip; single-stranded conformational polymorphism (SSCP) assay; restriction fragment length polymorphism (RFLP); automated fluorescent sequencing; clamped denaturing gel electrophoresis (CDGE); denaturing gradient gel electrophoresis (DGGE); mobility shift analysis; restriction enzyme analysis; heteroduplex analysis; chemical mismatch cleavage (CMC); RNase protection assays; use of polypeptides that recognize nucleotide mismatches; allele-specific PCR; sequence analysis; and SNP genotyping.

In some embodiments, genotype on an individual cat for SNP A1-212069607 may be determined using a method selected from the types of methods consisting of: hybridization-based methods, enzyme-based methods, post-amplification methods based on physical properties of DNA, and sequencing methods.

In some embodiments, genotype on an individual cat for SNP A1-212069607 may be determined using a method selected from the types of methods consisting of hybridization-based methods selected from the group consisting of: dynamic allele-specific hybridization, molecular beacon methods and SNP microarrays; enzyme-based methods selected from the group consisting of restriction fragment length polymorphism (RFLP), PCR-based methods, Flap endonuclease, primer extension methods, 5'- nuclease and oligonucleotide ligation assay; post-amplification methods based on physical properties of DNA selected from the group consisting of: single strand conformation polymorphism, temperature gradient gel electrophoresis, denaturing high performance liquid chromatography, high-resolution amplicon melting, DNA mismatch-binding proteins, SNPlex, and surveyor nuclease assay; and sequencing methods

In some embodiment, genotype on an individual cat for SNP A1-212069607 may be determined using a high-density array that contains genetic markers to interrogate SNP A1-212069607 for the presence of zero, one or two copies of the major and/or minor allele. In some embodiment, low-density array may be used. In some embodiment, in addition to interrogating the sample for the genotype with respect to SNP A1-212069607, other analyses and detections of SNPs may be performed using a high-density array containing genetic markers capable of detected a variety of SNPs.

Genotype analysis may be performed using hybridization-based methods. Examples of hybridization-based methods include dynamic allele-specific hybridization, methods that employ molecular beacons, and methods that employ SNP microarrays including high-density oligonucleotide SNP arrays or low-density oligonucleotide SNP arrays. SNPs can be interrogated by hybridizing complementary DNA probes to the SNP site. In dynamic allele-specific hybridization, a genomic segment is amplified and attached to a bead through a PCR reaction with a biotinylated primer. The amplified product is then attached to a streptavidin column and washed to remove the unbiotinylated strand. An allele-specific oligonucleotide is then added in the presence of a molecule that fluoresces when bound to double-stranded DNA. The intensity is measured as temperature is increased until the melting temperature (Tm) can be determined. SNP are detected by their lower than expected Tm. Specifically engineered single-stranded oligonucleotide probes are used in SNP detection that uses molecular beacons. Oligonucleotides are designed in which complementary regions are at each end and a probe sequence is located in between such that probe take on a hairpin, or stem-loop, structure in its natural, isolated state. A fluorophore is attached to one end of the probe a fluorescence quencher is attached to the other end. The fluorophore is in close proximity to the quencher when the oligo is in a hairpin configuration and the molecule does not emit fluorescence. The probe sequence is complementary to the genomic DNA used in the assay. If the probe sequence of the molecular beacon encounters its target genomic DNA during the assay, it will anneal and hybridize. The oligo will no longer assume the hairpin configuration and will fluoresce. High-density oligonucleotide SNP arrays comprise hundreds of thousands of probes arrayed on a small chip, allowing for many SNPs to be interrogated simultaneously. Several redundant probes designed to have the SNP site in several different locations as well as containing mismatches to the SNP allele are used to interrogate each SNP. The differential amount of hybridization of the target DNA to each of these redundant probes, allows for specific homozygous and heterozygous alleles to be determined.

Genotype analysis may be performed using enzyme-based methods. A broad range of enzymes including DNA ligase, DNA polymerase and nucleases may be employed. Examples of enzyme-based methods include methods based upon restriction fragment length polymorphism (RFLP), PCR-based methods, methods that utilize Flap endonuclease; methods that utilize primer extension, methods that utilize 5'- nuclease and methods that include oligonucleotide ligation assays. RFLP methods to detect SNPs use many different restriction endonucleases to digestion a genomic sample. It is possible to ascertain whether or not the enzymes cut the expected restriction sites by determining fragment lengths through a gel assay. RFLP assays are designed to include enzymes that cut in the presence or absence of SNPs and the pattern of fragment lengths can be used to determine the presence or absence of SNPs. PCR based methods include tetra-primer amplification refractory mutation system PCR, or ARMS-PCR, and multiple qPCR reactions. Tetra-primer amplification refractory mutation system PCR, or ARMS-PCR, employs two pairs of primers to amplify two alleles in one PCR reaction. The primers are designed such that the two primer pairs overlap at a SNP location but each match perfectly to only one of the possible SNPs. Alternatively, multiple qPCR reactions can be run with different primer sets that target each allele separately. Some embodiments utilize Flap endonuclease (FEN), which is an endonuclease that catalyzes structure-specific cleavage. This cleavage is highly sensitive to mismatches and can be used to interrogate SNPs with a high degree of specificity. A FEN called cleavase is combined with two specific oligonucleotide probes, that together with the target DNA, can form a tripartite structure recognized by cleavase. The first probe, called the Invader oligonucleotide is complementary to the 3' end of the target DNA. The last base of the Invader oligonucleotide is a non-matching base that overlaps the SNP nucleotide in the target DNA. The second probe is an allele-specific probe which is complementary to the 5' end of the target DNA, but also extends past the 3' side of the SNP nucleotide. The allele-specific probe will contain a base complementary to the SNP nucleotide.

Primer extension is a two-step process that first involves the hybridization of a probe to the bases immediately upstream of the SNP nucleotide followed by a 'mini-sequencing' reaction, in which DNA polymerase extends the hybridized primer by adding a base that is complementary to the SNP nucleotide. This incorporated base is detected and determines the SNP allele. The primer extension method is used in a number of assay formats. These formats use a wide range of detection techniques that include MALDI-TOF Mass spectrometry (see Sequenom) and ELISA-like methods. Sequenom's iPLEX SNP genotyping method, which uses a MassARRAY mass spectrometer. The flexibility and specificity of primer extension make it amenable to high throughput analysis. Primer extension probes can be arrayed on slides allowing for many SNPs to be genotyped at once. Referred to as arrayed primer extension (APEX), this technology has several benefits over methods based on differential hybridization of probes.

Several methods of genotype analysis are based upon DNA's physical properties such as melting temperature and single stranded conformation. Methods that use single stranded conformation are based upon single-stranded DNA (ssDNA) that folds into a tertiary structure. The conformation is sequence dependent and most single base pair mutations will alter the shape of the structure. When applied to a gel, the tertiary shape will determine the mobility of the ssDNA, providing a mechanism to differentiate between SNP alleles. This method first involves PCR amplification of the target DNA. The double-stranded PCR products are denatured using heat and formaldehyde to produce ssDNA. The ssDNA is applied to a non-denaturing electrophoresis gel and allowed to fold into a tertiary structure. Differences in DNA sequence will alter the tertiary conformation and be detected as a difference in the ssDNA strand mobility. Temperature gradient gel electrophoresis (TGGE) or temperature gradient capillary electrophoresis (TGCE) methods are based on the principle that partially denatured DNA is more restricted and travels slower in a gel or other porous material. In another method, denaturing high performance liquid chromatography (DHPLC) uses reversed-phase HPLC to interrogate SNPs. In DHPLC, the solid phase which has differential affinity for single and double-stranded DNA. Another method used is high-resolution melting of the entire amplicon. Use of DNA mismatch-binding proteins may also be used to detect SNPs. MutS protein from Thermus aquaticus binds different single nucleotide mismatches with different affinities and can be used in capillary electrophoresis to differentiate all six sets of mismatches. SNPlex is a proprietary genotyping platform sold by Applied Biosystems. Surveyor nuclease assay uses surveyor nuclease, a mismatch endonuclease enzyme that recognizes all base substitutions and small insertions/deletions (indels), and cleaves the 3' side of mismatched sites in both DNA strands. Sequencing technologies can also be used in SNP detection. Advances in sequencing technology allow SNP detection by sequencing more practical.

Genotyping by sequencing using next generation sequencing technologies has become a common practice. Genotyping by sequencing, also called GBS, is a method to discover single nucleotide polymorphisms (SNP) in order to perform genotyping studies, such as genome-wide association studies (GWAS). GBS uses restriction enzymes to reduce genome complexity and genotype multiple DNA samples. After digestion, PCR is performed to increase fragments pool and then GBS libraries are sequenced using next generation sequencing technologies. With the advancement of next generation sequencing technologies such as Illumina short read sequencing by synthesis and PacBio's single molecule real time sequencing it is becoming more feasible to do GBS. In the future, development of new technologies such as nanopore single molecule sequencing may allow whole genome sequencing/genotyping.

### BAIB Concentration

As noted above, BAIB concentrations have an association with genotype. Cats with the AA genotype have the highest BAIB concentrations compared cats with the AG and GG genotypes and cats with the AG genotype have higher BAIB concentrations compared cats with the GG genotypes. As noted above, feline subjects with the AA and AG genotypes have an increased risk or increased likelihood of developing calcium oxalate stones compared to cats with the GG genotype. Accordingly, feline subjects with higher BAIB concentrations, such as those found in feline subjects with the AA and AG genotypes have an increased risk or increased likelihood of developing calcium oxalate stones compared to cats with the lower BAIB concentrations of the GG genotype. BAIB levels may be used as a surrogate marker for identifying feline subjects with increased risk or increased likelihood of developing calcium oxalate stones and for identifying feline subjects that do not have increased risk or increased likelihood of developing calcium oxalate stones. BAIB levels may be used as a surrogate marker for identifying feline subjects who would benefit from treatment involving a low arginine diet to reduce the likelihood of developing calcium oxalate stones and for identifying feline subjects who would not benefit from treatment involving a low arginine diet to reduce the likelihood of developing calcium oxalate stones. Methods to reduce the likelihood that a feline subject would develop calcium oxalate stones may comprise identifying the feline subjects as a cat that would benefit from treatment involving a low arginine diet to reduce the likelihood of developing calcium oxalate stones by detecting of BAIB concentrations in the feline subject and comparing them to a positive reference value or positive control. A feline subject identified as a cat that would benefit from the treatment would then be fed a diet low in arginine.

BAIB can be detected in any one of the following samples using the following methods.

Tables 1 and 2 provide mean BAIB concentrations for the various genotypes. Such information can be used to establish a positive reference BAIB concentration value. Detectable BAIB concentration in a sample from a feline subject that is equal to or greater than the positive reference BAIB concentration value indicates that the feline subject would benefit from treatment with a low arginine diet to reduce the likelihood that a feline subject would develop calcium oxalate stones. The positive reference BAIB concentration value would act as a threshold wherein detected levels equal to or greater than it would indicate that the feline subject would benefit from the treatment with a low arginine diet to reduce the likelihood of developing calcium oxalate stones and detected levels less than it would indicate that the feline subject would not benefit from the treatment with a low arginine diet to reduce the likelihood of developing calcium oxalate stones. A negative reference BAIB concentration value could also be established to act as a threshold wherein detected levels equal to or less than such negative reference BAIB concentration value would indicate that the feline subject would not benefit from the treatment with a low arginine diet to reduce the likelihood of developing calcium oxalate stones. As additional data is generated for more subjects, the precision and accuracy of identifying the positive reference BAIB concentration value and any negative reference BAIB concentration value will continually be fine-tuned.

In addition to using the data in Tables 1 and 2 to establish a positive reference BAIB concentration value, the data can also be used to prepare positive control BAIB concentration samples. Positive control samples could be biological samples spiked with BAIB at a concentration corresponding to the threshold concentration to determine whether a feline subject would benefit from the treatment with a low arginine diet to reduce the likelihood of developing calcium oxalate stones or would not benefit. The BAIB concentration would preferably be determined for the sample from the feline subject and the positive control to render the outcome more reliable. Detected levels in the sample from the feline subject equal to or greater than the positive control indicate that the feline subject would benefit from the treatment with a low arginine diet to reduce the likelihood of developing calcium oxalate stones. Detected levels in the sample from the feline subject less than the positive control indicate that the feline subject would not benefit from the treatment with a low arginine diet to reduce the likelihood of developing calcium oxalate stones. A negative control BAIB concentration sample could also be prepared to serve as a negative control with a threshold concentration wherein detected levels equal to or less than the BAIB concentration in the negative control BAIB concentration sample would indicate that the feline subject would not benefit from the treatment with a low arginine diet to reduce the likelihood of developing calcium oxalate stones. Negative controls samples can be prepared by spiking a specimen such as a biological sample having no BAIB. As additional data is generated for more subjects, the precision and accuracy of identifying the positive reference BAIB concentration values and any negative reference BAIB concentration value will continually be fine-tuned and such information used to improve positive control BAIB concentration samples and negative control BAIB concentration samples.

Methods of treatment to reduce the likelihood of developing calcium oxalate stones can comprise identifying a feline subject as a cat that would benefit from the treatment with a low arginine diet to reduce the likelihood of developing calcium oxalate stones using detection of BAIB concentration in a sample from the feline subject, comparing the detected level to a positive reference value or the results of a positive control assay in which BAIB concentration is measured in a positive control BAIB concentration sample. A feline subject identified as a cat that would benefit from the treatment with a low arginine diet to reduce the likelihood of developing calcium oxalate stones using such methods is fed a low arginine diet to reduce the likelihood of developing calcium oxalate stones.

### 2-oxoarginine Concentration

In addition to BAIB concentrations, 2-oxoarginine concentrations have an association with genotype. Cats with the AA genotype have the lowest 2-oxoarginine concentrations compared cats with the AG and GG genotypes and cats with the AG genotype have lower 2-oxoarginine concentrations compared cats with the GG genotypes (See Table 16). As noted above, feline subjects with the AA and AG genotypes have an increased risk or increased likelihood of developing calcium oxalate stones compared to cats with the GG genotype. Accordingly, feline subjects with lower 2-oxoarginine concentrations, such as those found in feline subjects with the AA and AG genotypes have an increased risk or increased likelihood of developing calcium oxalate stones compared to cats with the higher 2-oxoarginine concentrations of the GG genotype. 2-oxoarginine levels may be used as a surrogate marker for identifying feline subjects with increased risk or increased likelihood of developing calcium oxalate stones and for identifying feline subjects that do not have increased risk or increased likelihood of developing calcium oxalate stones. 2-oxoarginine levels may be used as a surrogate marker for identifying feline subjects who would benefit from treatment involving a low arginine diet to reduce the likelihood of developing calcium oxalate stones and for identifying feline subjects who would not benefit from treatment involving a low arginine diet to reduce the likelihood of developing calcium oxalate stones. Methods to reduce the likelihood that a feline subject would develop calcium oxalate stones may comprise identifying the feline subjects as a cat that would benefit from treatment involving a low arginine diet to reduce the likelihood of developing calcium oxalate stones by detecting of 2-oxoarginine concentrations in the feline subject and comparing them to a positive reference value or positive control. A feline subject identified as a cat that would benefit from the treatment would then be fed a diet low in arginine.

2-oxoarginine can be detected in samples using the well-known methods. Tables 16 and 2 provide mean 2-oxoarginine concentrations for the various genotypes. Such information can be used to establish a positive reference 2-oxoarginine concentration value. Detectable 2-oxoarginine concentration in a sample from a feline subject that is equal to or less than the positive reference 2-oxoarginine concentration value indicates that the feline subject would benefit from treatment with a low arginine diet to reduce the likelihood that a feline subject would develop calcium oxalate stones. The positive reference 2-oxoarginine concentration value would act as a threshold wherein detected levels equal to or less than it would indicate that the feline subject would benefit from the treatment with a low arginine diet to reduce the likelihood of developing calcium oxalate stones. A negative reference 2-oxoarginine concentration value could also be established to act as a threshold wherein detected levels equal to or greater than such negative reference 2-oxoarginine concentration value would indicate that the feline subject would not benefit from the treatment with a low arginine diet to reduce the likelihood of developing calcium oxalate stones. As additional data is generated for more subjects, the precision and accuracy of identifying the positive reference 2-oxoarginine concentration value and any negative reference 2-oxoarginine concentration value will continually be fine-tuned.

In addition to using the data in Table 16 to establish a positive reference 2-oxoarginine concentration value, the data can also be used to prepare positive control 2-oxoarginine concentration samples. Positive control samples could be biological samples spiked with 2-oxoarginine at a concentration corresponding to the threshold concentration to determine whether a feline subject would benefit from the treatment with a low arginine diet to reduce the likelihood of developing calcium oxalate stones or would not benefit. The 2-oxoarginine concentration would preferably be determined for the sample from the feline subject and the positive control to render the outcome more reliable. Detected levels in the sample from the feline subject equal to or less than the positive control indicate that the feline subject would benefit from the treatment with a low arginine diet to reduce the likelihood of developing calcium oxalate stones. A negative control 2-oxoarginine concentration sample could also be prepared to serve as a negative control with a threshold concentration wherein detected levels equal to or less than the 2-oxoarginine concentration in the negative control 2-oxoarginine concentration sample would indicate that the feline subject would not benefit from the treatment with a low arginine diet to reduce the likelihood of developing calcium oxalate stones. Negative controls samples can be prepared by spiking a specimen such as a biological sample having no 2-oxoarginine. As additional data is generated for more subjects, the precision and accuracy of identifying the positive reference 2-oxoarginine concentration values and any negative reference 2-oxoarginine concentration value will continually be fine-tuned and such information used to improve positive control 2-oxoarginine concentration samples and negative control 2-oxoarginine concentration samples.

Methods of treatment to reduce the likelihood of developing calcium oxalate stones can comprise identifying a feline subject as a cat that would benefit from the treatment with a low arginine diet to reduce the likelihood of developing calcium oxalate stones using detection of 2-oxoarginine concentration in a sample from the feline subject, comparing the detected level to a positive reference value or the results of a positive control assay in which 2-oxoarginine concentration is measured in a positive control 2-oxoarginine concentration sample. A feline subject identified as a cat that would benefit from the treatment with a low arginine diet to reduce the likelihood of developing calcium oxalate stones using such methods is fed a low arginine diet to reduce the likelihood of developing calcium oxalate stones.

### Compositions and formulations

Application of the methodology outlined above has identified bioactive dietary components that have been combined to provide compositions, foods, and diets that provide significant benefits to cats identified that will benefit from a treatment to reduce the likelihood of calcium oxalate stone formation. The risk of calcium oxalate stone formation in feline subjects with the AA or AG genotype can be significantly reduced by feeding a low arginine diet.

The food product is a nutritionally complete diet for an adult feline. In a specific aspect, the food product is a nutritionally complete diet formulated for an adult companion feline.

In some embodiments, the compositions include food compositions contain less than 1.84% or less of arginine based on the total weight of the composition on a dry matter basis. In some embodiments, the compositions include food compositions contain 1.04-1.84% arginine based on the total weight of the composition on a dry matter basis. In some embodiments, the compositions include food compositions contain 1.21% or less of arginine based on the total weight of the composition on a dry matter basis. In some embodiments, the compositions include food compositions contain 1.11% or less of arginine based on the total weight of the composition on a dry matter basis. In some embodiments, the compositions include food compositions contain less than 1.04% arginine based on the total weight of the composition on a dry matter basis. The compositions may comprise protein in an amount from 4% to 75% or more based on the total weight of the composition on a dry matter basis, fat in an amount from 5% to 50% or more based on the total weight of the composition on a dry matter basis, and carbohydrate from 5% to 75% or more based on the total weight of the composition on a dry matter basis, wherein the food composition is suitable for consumption by a cat.

In some embodiments, a nutritionally complete and balanced low arginine (i.e.,) cat food composition may comprise, based on the total weight of the composition on a dry matter basis, 1.84% or less arginine, or 1.21% or less of arginine, or 1.11% or less of arginine or less than 1.04% arginine, or between 1.04-1.84% arginine, and may further comprise from 4% to 90%, from 5% to 75%, from 10% to 60% protein, and from 15% to 50% by weight of protein; from 0% to 90%, from 2% to 80%, from 5% to 75%, and from 10% to 50% by weight of carbohydrate; from 2% to 60%, from 5% to 50%, and from 10% to 35% by weight of fat. The compositions may further contain from 0 to 15% or from 2% to 8%, by weight of vitamins and minerals, antioxidants, and other nutrients which support the nutritional needs of the animal.

Sources of proteins, carbohydrates, fats, vitamins, minerals, balancing agents, and the like, suitable for inclusion in the compositions, and particularly in the food products to be administered in methods disclosed but not claimed herein, may be selected from among those conventional materials known to those of ordinary skill in the art.

In some embodiments, proteins useful as ingredients of the food compositions may comprise proteins from animal sources, such as animal proteins, including mammalian, avian protein, reptilian, amphibian, fish, invertebrate proteins and combinations thereof; e.g., from any of cattle, sheep, pig, goat, deer, rabbit, horse, kangaroo, their milk, curds, whey or blood, and internal tissues and organs such as smooth muscle, striate muscle, liver, kidney, intestine or heart; additional avian protein sources encompass turkey, goose, duck, ostrich, quail, pigeon, their eggs and internal tissues and organs such as smooth muscle, striate muscle, liver, kidney, intestine or heart; amphibian sources include frog or salamander, reptilian protein sources include alligator, lizard, turtle and snake; a fish protein sources include catfish, herring, salmon, tuna, bluefish, cod, halibut, trout, swordfish and their eggs; and an invertebrate protein sources include lobster, crab, clams, mussels or oysters, and combinations thereof, meat protein isolate, whey protein isolate, egg protein, mixtures thereof, and the like, as well as vegetable sources, such as soy protein isolate, corn gluten meal, wheat gluten, mixtures thereof, and the like.

In some embodiments, carbohydrates useful as ingredients of the food compositions may include but are not limited to, one or more of corn, whole yellow corn, grain sorghum, wheat, barley, rice, millet, brewers rice, oat groats, and polysaccharides (e.g., starches and dextrins) and sugars (e.g., sucrose, lactose, maltose, glucose, and fructose) that are metabolized for energy when hydrolyzed. Examples of additional carbohydrate sources suitable for inclusion in the compositions disclosed herein include, fruits and non-tomato pomace vegetables.

Fats useful as ingredients of the food compositions may be from any source, such as but not limited to poultry fat, beef tallow, lard, choice white grease, soybean oil, corn oil, canola oil, sunflower oil, mixtures thereof, and the like. The fat may be incorporated completely within the food composition, deposited on the outside of the food composition, or a mixture of the two methods.

In some embodiments, the compositions further include an effective amount of one or more substances selected from the group consisting of glucosamine, chondroitin, chondroitin sulfate, methylsulfonylmethane ("MSM"), creatine, antioxidants, Perna canaliculata, omega-3 fatty acids, omega-6 fatty acids and mixtures thereof.

In some embodiments, the food composition further comprises one or more amino acid such as but not limited to arginine, histidine, isoleucine, leucine, lysine, methionine (including DL-methionine, and L-methionine), phenylalanine, threonine, tryptophan, valine, taurine, carnitine, alanine, aspartate, cystine, glutamate, glutamine, glycine, proline, serine, tyrosine, and hydroxyproline.

In some embodiments, the food composition further comprises one or more fatty acids such as but not limited to lauric acid, myristic acid, palmitic acid, palmitoleic acid, margaric acid, margaroleic acid, stearic acid, oleic acid, linoleic acid, g-linolenic acid, a-linolenic acid, stearidonic acid, arachidic acid, gadoleic acid, DHGLA, arachidonic acid, eicossatetra acid, EPA, behenic acid, erucic acid, docosatetra acid, and DPA.

In some embodiments, the food composition further comprises one or more macro nutrients such as but not limited to moisture, protein, fat, crude fiber, ash, dietary fiber, soluble fiber, insoluble fiber, raffinose, and stachyose.

In some embodiments, the food composition further comprises one or more micro nutrients such as but not limited to beta-carotene, alpha-lipoic acid, glucosamine, chondroitin sulfate, lycopene, lutein, and quercetin.

In some embodiments, the food composition further comprises one or more minerals such as but not limited to calcium, phosphorus, potassium, sodium, chloride, iron, copper, copper, manganese, zinc, iodine, selenium, selenium, cobalt, sulfur, fluorine, chromium, boron, and oxalate.

In some embodiments, the food composition further comprises one or more other vitamins, such as but not limited to vitamin A, vitamin C, vitamin D, vitamin E, quinoa grain, thiamine, riboflavin, niacin, pyridoxine, pantothenic acid, folic acid, vitamin B12, biotin, and choline.

In some embodiments, the food composition further comprises fiber, which may be supplied from a variety of sources, including, for example, vegetable fiber sources such as cellulose, beet pulp, peanut hulls, and soy fiber.

In some embodiments, the food composition further comprises stabilizing substances, for example, substances that tend to increase the shelf life of the composition. Potentially suitable examples of such substances include, for example, preservatives, antioxidants, synergists and sequestrants, packaging gases, stabilizers, emulsifiers, thickeners, gelling agents, and humectants. Examples of emulsifiers and/or thickening agents include, for example, gelatin, cellulose ethers, starch, starch esters, starch ethers, and modified starches.

In some embodiments, the food composition further comprises additives for coloring, palatability, and nutritional purposes include, for example, colorants; iron oxide, sodium chloride, potassium citrate, potassium chloride, and other edible salts; vitamins; minerals; and flavoring. The amount of such additives in a composition typically is up to 5% (dry basis of the composition).

### Preparation of Compositions

The low arginine compositions may be prepared as food products suitable for consumption by cats. These food products may be of any consistency or moisture content; i.e., the compositions may be moist, semi-moist, or dry food products. "Moist" food products are generally those with a moisture content of from 60% to 90% or greater. "Dry" food products are generally those with a moisture content of from 3% to 11%, and are often manufactured in the form of small pieces or kibbles. "Semi-moist food products generally have a moisture content of from 25% to 35%. The food products may also include components of more than one consistency, for example, soft, chewy meat-like particles or pieces as well as kibble having an outer cereal component or coating and an inner "cream" component.

The low arginine food products may be prepared in a canned or wet form using conventional food preparation processes known to those of ordinary skill in the art. Typically, ground animal proteinaceous tissues are mixed with the other ingredients, such as cereal grains, suitable carbohydrate sources, fats, oils, and balancing ingredients, including special purpose additives such as vitamin and mineral mixtures, inorganic salts, cellulose, beet pulp and the like, and water in an amount sufficient for processing. The ingredients are mixed in a vessel suitable for heating while blending the components. Heating the mixture is carried out using any suitable manner, for example, direct steam injection or using a vessel fitted with a heat exchanger. Following addition of all of the ingredients of the formulation, the mixture is heated to a temperature of from 50° F. to 212° F. Although temperatures outside this range can be used, they may be commercially-impractical without the use of other processing aids. When heated to the appropriate temperature, the material will typically be in the form of thick liquid, which is dispensed into cans. A lid is applied and the container is hermetically sealed. The sealed can is then placed in convention equipment designed for sterilization of the contents. Sterilization is usually accomplished by heating to temperatures of greater than 230° C. for an appropriate time depending on the temperature used, the nature of the composition, and related factors. The compositions and food products of the present invention can also be added to or combined with food compositions before, during, or after their preparation.

In some embodiments, the food products may be prepared in a dry form using convention processes known to those of ordinary skill in the art. Typically, dry ingredients, including dried animal protein, plant protein, grains and the like are ground and mixed together. Liquid or moist ingredients, including fats, oils water, animal protein, water, and the like are added combined with the dry materials. The specific formulation, order of addition, combination, and methods and equipment used to combine the various ingredients can be selected from those known in the art. For example, in certain embodiments, the resulting mixture is process into kibbles or similar dry pieces, which are formed using an extrusion process in which the mixture of dry and wet ingredients is subjected to mechanical work at high pressure and temperature, forced through small openings or apertures, and cut off into the kibbles, e.g., with a rotating knife. The resulting kibble can be dried and optionally coated with one or more topical coatings comprising, e.g., flavors, fats, oils, powdered ingredients, and the like. Kibbles may also be prepared from dough by baking, rather than extrusion, in which the dough is placed into a mold before dry-heat processing.

In preparing a composition, any ingredient generally may be incorporated into the composition during the processing of the formulation, e.g., during and/or after mixing of the other components of the composition. Distribution of these components into the composition can be accomplished by conventional means. In certain embodiments, ground animal and/or poultry proteinaceous tissues are mixed with other ingredients, including nutritional balancing agents, inorganic salts, and may further include cellulose, beet pulp, bulking agents and the like, along with sufficient water for processing.

In some embodiments, the compositions are formulated so as to be easier to chew. In specific embodiments, the compositions and food products are formulated to address specific nutritional differences between cats such as life stage, age, size, weight, body composition, and breed.

The low arginine compositions are formulated as a nutritionally complete diet to meet the needs of a mature adult feline. These nutritionally complete diets that include sufficient nutrients for maintenance of normal health of a healthy cat on the diet. Nutritionally complete and balanced cat food compositions are familiar to one of skill in the art. For example, substances such as nutrients and ingredients suitable for nutritionally complete and balanced animal feed compositions, and recommended amounts thereof, may be found for example, in the Official Publication of the Association of American Feed Control Officials. Inc. (AAFCO), Atlanta, Ga. (2012).

All publications mentioned herein are describing and disclosing the materials and methodologies that are reported in the publication, which might be used in connection with the invention.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention.

### EXAMPLES

### Example 1

A study was completed, where cats with the AG genotype were compared to cats with a homozygous GG genotype. The study used 34 cats and compared three foods. All foods were fed for one month and at the end of the one month feeding period the cats' blood was analyzed for BAIB. Each cat consumed each food, so that each cat had a blood sample from each food after a one month feeding period.

Table 3 show the dietary concentration (as percent) of fiber and arginine.

**TABLE 3**

| Diet | Fiber | Arginine |
|---|---|---|
| Increased fiber, high arginine | 1.4 | 1.84 |
| Increased fiber, low arginine | 1.3 | 1.47 |
| Low fiber, low arginine | 0.9 | 1.44 |

Table 4 shows the BAIB concentration in relative terms for the cats in the study. Each genotype had 17 cats.

**TABLE 4**

| Diet | AG genotype | GG genotype |
|---|---|---|
| Increased fiber, high arginine | 4.7 | 1.6 |
| Increased fiber, low arginine | 3.4 | 1.5 |
| Low fiber, low arginine | 3.7 | 1.6 |

As shown in Table 5, the differential effect of food on cats having the different genotypes was especially apparent in the stone forming cats.

**TABLE 5**

| Genotype | AG Genotype | | GG Genotype | |
|---|---|---|---|---|
| Stones | No | Yes | No | Yes |
| Number of cats | N=11 | N=6 | N=16 | N=1 |
| Increased fiber, high arginine | 3.6 | 6.6 | 1.6 | 1.0 |
| Increased fiber, low arginine | 2.8 | 4.3 | 1.6 | 1.0 |
| Low fiber, low arginine | 3.4 | 4.4 | 1.7 | 0.9 |

There is a significant (P<0.01) decline in the AG genotype and a significant difference between the genotypes (p=0.02) in their change in BAIB concentration. These data show that in the cats with the AG genotype, there is a significant benefit in reducing BAIB, which is a biomarker of calcium oxalate stone risk, through reduction of the dietary amino acid arginine.

### Example 2

Blood is collected in order to determine plasma metabolomic profiles. Levels of BAIB and/or 2-oxarginine in plasma can be measured by a commercial laboratory (Metabolon, Durham, NC, USA). Extracted supernatant is split and run on gas chromatography and liquid chromatography mass spectrometer platforms. The peak for each of BAIB and 2-oxarginine is known and the area under the peak for each sample can be normalized to a known sample. (See e.g. Evans, A.M., et al. (2009). Integrated, nontargeted ultrahigh performance liquid chromatography/electrospray ionization tandem mass spectrometry platform for the identification and relative quantification of the small-molecule complement of biological systems. Anal. Chem. 81, 6656-6667.) Gas chromatography (for hydrophobic molecules) and liquid chromatography (for hydrophilic molecules) are used to identify and provide relative quantification of metabolites such as BAIB present in plasma samples. (See e.g.: Ballet, C. et al. (2018) New enzymatic and mass spectrometric methodology for the selective investigation of gut microbiota-derived metabolites, Chem. Sci., 9, 6233-6239; Akiyama, Y et al. (2012) A Metabolomic Approach to Clarifying the Effect of AST-120 on 5/6 Nephrectomized Rats by Capillary Electrophoresis with Mass Spectrometry (CE-MS) Toxins 2012, 4(11), 1309-1322; and Kikuchi K, et al. (2010) Metabolomic search for uremic toxins as indicators of the effect of an oral sorbent AST-120 by liquid chromatography/tandem mass spectrometry. J Chromatogr B Analyt Technol Biomed Life Sci 878:2997-3002.) Burrage, LC et al. (2019) Untargeted metabolomic profiling reveals multiple pathway perturbations and new clinical biomarkers in urea cycle disorders, Genetics in Medicine 21, 1977-1986 disclose analysis of plasma samples including measurement of metabolite levels. Miller MJ, et al. (2015) Untargeted metabolomic analysis for the clinical screening of inborn errors of metabolism. J Inherit Metab Dis. 38:1029-1039 and Kennedy AD, et al. (2016) Metabolomic profiling of human urine as a screen for multiple inborn errors of metabolism. Genet Test Mol Biomarkers 20:485-495 also disclose technologies for measuring 2-oxoargine. Techniques may be adapted and used to measure metabolite levels in samples from a feline subject. Midttun, O., et al. (2013). High-throughput, low-volume, multianalyte quantification of plasma metabolites related to one-carbon metabolism using HPLC-MS/MS. Anal Bioanal Chem 405, 2009-017, Fernàndez-Roig, S., (2013) Low folate status enhances pregnancy changes in plasma betaine and dimethylglycine concentrations and the association between betaine and homocysteine. Am J Clin Nutr 97, 1252-59 and Holm, P.I., et al. (2005). Betaine and folate status as cooperative determinants of plasma homocysteine in humans. Arterioscler Thromb Vase Biol 25, 379-385 disclose technologies for measuring betaine. Techniques may be adapted and used to measure betaine levels in samples from a feline subject.

### Example 3

A saliva sample is obtained from a feline. The sample may be shipped as collected to a laboratory at another location, partially processed and then shipped to a laboratory at another location or completely processed and analyzed at a laboratory and the site of collection. If the sample is shipped as collected to a laboratory at another location or partially processed and then shipped to a laboratory at another location, results which may include some or all data collected from the sample by the laboratory may be transmitted to the site of collection and/or a veterinarian and/or the owner of or responsible party for the feline. After the saliva sample is obtained, it may be processed for analysis and evaluated for the presence of the AA and AG genotype.

If results indicate that the feline will benefit from the treatments disclosed but not claimed herein, the feline may be fed a low arginine diet.

### Example 4

Samples are collected from felines using PERFORMAgene PG-100 Oral collection kit.

When doing so, the animal should not eat for 30 minutes or drink for 10 minutes before saliva collection, the individual doing the collection should not scrape the animal's teeth or cheek with the sponge nor should the animal be allowed to chew or bite the sponge.

The collection tube provided as part of the PERFORMAgene PG-100 Oral collection kit contains liquid that preserves the DNA sample and is required by the lab to analyze the sample. The cap should not be removed prior to sample collection.

In the first step of the collection protocol, the sponge is placed in the animal's mouth at the cheek pouch. Saliva is collected for 30 seconds by moving sponge and mopping saliva where it naturally pools (in the cheek pouch and under the tongue). For animals older than 6 months, moderate restraint may be required.

Next, holding the tube upright, the cap from the tube is unscrewed. The cap is turned upside down and the oral swab is placed in the tube. The cap is screwed on tightly to prevent liquid sample from leaking during transport. The tube is inverted and shaken vigorously numerous times, e.g. 10 times, to thoroughly mix sample.

A permanent marker may be used to clearly write the animal identification number on the white space available on the tube label.

The step-by-step laboratory protocol for manual purification of DNA from 0.5 mL aliquot of a Performagene^{™} sample that has been collected and preserved in Performagene chemistry with the PG-100 collection kit is as follows. The Reagents required for manual purification are available with PG-AC1 reagent package or PG-AC4 reagent package.

When a DNA sample is collected and mixed with the Performagene solution, the DNA is immediately stabilized Performagene samples are stable at room temperature for 1 year from the time of collection. Performagene samples can be stored indefinitely at -15°C to -20°C, and can undergo multiple freeze-thaw cycles without deterioration of the DNA.

The following equipment and reagents are used in the purification process: a Microcentrifuge capable of running at 15,000 × g; an air or water incubator at 50°C; ethanol (95% to 100%) at room temperature; DNA buffer: TE (10 mM Tris-HCl, 1mM EDTA, pH 8.0) or similar solution; optional glycogen (20 mg/mL) (e.g., Invitrogen Cat. No. 10814-010); ethanol (70%) at room temperature and 5M NaCl solution.

In the first step, the sample is mixed by shaking vigorously for 5 seconds. This is to ensure that viscous samples are properly mixed with the Performagene solution.

The sample is incubated in a 50°C air incubator for a minimum of 2 hours, or in a 50°C water incubator for a minimum of 1 hour. DNA in Performagene is stable at room temperature even without the incubation step. This heat-treatment step is essential to ensure that DNA is adequately released and that nucleases are permanently inactivated. This incubation step may be performed at any time after sample is collected from the animal and before it is purified. Incubation of the entire sample is recommended. The sample may be incubated at 50°C overnight if it is more convenient. A longer time is required in an air incubator because temperature equilibration is slower than in a water incubator.

Optionally, the collection sponge may be removed. The cap is removed and the collection sponge is pressed against the inside of the tube to extract as much of the sample as possible. The sponge and cap are discarded. Sponge removal is dictated by preference of workflow.

Next, 500 µL of the mixed Performagene sample is transferred to a 1.5 mL microcentrifuge tube. The remainder of the Performagene sample can be stored at room temperature or frozen (-15°C to -20°C). 20 µL (1/25th volume) of PG-L2P purifier is then added to the microcentrifuge tube and mix ed by vortexing for a few seconds. The sample becomes turbid as impurities and inhibitors are precipitated.

The sample is incubated on ice for 10 minutes (room temperature incubation can be substituted but will be slightly less effective in removing impurities) followed by centrifugation at room temperature for 5 minutes at 15,000 × g. A longer period of centrifugation (up to 15 minutes) may be beneficial in reducing the turbidity (high A320) of the final DNA solution. The clear supernatant is transferred with a pipette tip into a fresh microcentrifuge tube and the pellet, which contains turbid impurities, is discarded. To 500 µL of supernatant, 25 µL (1/20^{th} volume) of 5 M NaCl is added followed by mixing. The addition of NaCl is necessary to ensure efficient recovery of DNA. To 500 µL of supernatant, 600 µL of room temperature 95% to 100% ethanol is added followed by gentle mixing by inversion 10 times. During mixing with ethanol, the DNA will be precipitated. The DNA may appear as a clot of DNA fibers or as a fine precipitate, depending upon the amount of DNA in the sample. Even if no clot is seen, DNA will be recovered by carefully following the next steps.

The sample is allowed to stand at room temperature for 10 minutes to allow the DNA to fully precipitate. The tube is then placed in the centrifuge in a known orientation (DNA pellet may not be visible after centrifugation) and centrifuged at room temperature for 2 minutes at >15,000 × g. For example, each tube may be placed in the microcentrifuge with the hinge portion of the cap pointing away from the center of the rotor. After centrifugation, the position of the pellet can be located (even if too small to be easily visible); it will be at the tip of the tube below the hinge.

The supernatant is removed with a pipette tip and discarded. The pellet contains DNA. Rotating the tube such that the pellet is on the upper wall will allow you to safely move a pipette tip along the lower wall and remove all of the supernatant. The supernatant may contain impurities and should be removed as completely as possible. Excessive drying of the pellet can make the DNA more difficult to dissolve. The DNA is washed by first adding 250 µL of 70% ethanol, then letting it stand for 1 minute at room temperature. The ethanol is removed with a pipette tip without disturbing the pellet. The 70% ethanol wash helps to remove residual inhibitors. Complete removal of ethanol, however, is essential to prevent inhibition during downstream applications. Therefore, the tube is centrifuged for 6 seconds to pool any remaining ethanol, which is removed with a pipette tip.

100 µL of DNA buffer (e.g. TE buffer) is added to the tube to dissolve the DNA pellet. Vortexing for at least 5 seconds aids in the dissolving process. To ensure complete rehydration of the DNA, let sit at room temperature overnight. DNA can now be quantified and used in downstream applications.

Assays that use fluorescent dyes are more specific than absorbance at 260 nm for quantifying the amount of double-stranded DNA (dsDNA) in a DNA sample. To quantify the DNA by fluorescence method, fluorescent dyes such as PicoGreen^{®} or SYBR^{®} Green I may be used to quantify dsDNA since there is less interference by contaminating RNA. Alternatively, commercially available kits such as Invitrogen's Quant-iT^{™} PicoGreen dsDNA Assay Kit (Cat. No. Q-33130) can be used. For either protocol, the purified DNA is preferably diluted 1:50 with TE solution and 5 µL is used in the quantification assay.

Alternatively, DNA may be quantified by absorbance in which case the purified sample is preferable first treated with RNase to digest contaminating RNA and then remove the RNA fragments by ethanol precipitation of the DNA. DNA from a Performagene sample typically contains appreciably more RNA than found in blood samples. Ensure that alcohol-precipitated DNA is fully dissolved before reading the absorbance. An absorbance of 1.0 at 260 nm corresponds to a concentration of 50 ng/µL (50 µg/mL) for pure dsDNA. A spectrophotometer cuvette capable of reading a volume of 100 µL or less should be used to avoid using too large a volume of sample. Absorbance values at 260 nm should be between 0.1 and 1.5. Lower values may not be reliable.

A 10 µL aliquot of purified RNase-treated DNA is diluted with 90 µL of TE (1/10 dilution) and mixed by gently pipetting up and down. Wait for bubbles to clear. TE is used in the reference (blank) cell. The absorbance is measured at 320 nm, 280 nm and 260 nm. Corrected A₂₈₀ and A₂₆₀ values are calculated by subtracting the absorbance at 320 nm (A₃₂₀) from A₂₈₀ and A₂₆₀ values. DNA concentration in ng/µL = corrected A₂₆₀ × 10 (dilution factor) × 50 (conversion factor). A₂₆₀/A₂₈₀ ratio: divide corrected A₂₆₀ by corrected A₂₈₀.

### Example 5

A daily diet that reduces the likelihood of calcium oxalate stones in cats identified as having a higher likelihood or risk of developing calcium oxalate stones is disclosed but not claimed. In some embodiments, methods may comprise feeding a low arginine diet to a cat identified as having the AA or AG genotype of SNP A1_212891692. In some embodiments, methods may comprise feeding low arginine diet to a cat identified as having BAIB levels that are equal to or greater than a reference level corresponding to levels in cats identified as benefiting from the treatment and/or equal to or greater than levels measured in a positive control sample which contains BAIB at a level corresponding to levels in cats identified as benefiting from the treatment. In some embodiments, methods may comprise analyzing a sample from a feline subject to determine if the feline subject has the AA or AG genotype of SNP A1_212891692. In some embodiments, methods may comprise analyzing a sample from a feline subject to determine the level of BAIB in the sample and comparing the measured level to either a reference level corresponding to a positive test result and/or the level measured in a positive control sample. The positive reference standard value corresponds to BAIB levels deemed to be a positive result for a cat of comparable size, weight, age, breed inter alia.

### Example 6

The following composition is based upon total nutrition to be provided per day. In some embodiments based on the total weight of the composition on a dry matter basis, the amount of arginine is equal to 1.84% or less. In some embodiments based on the total weight of the composition on a dry matter basis, the amount of arginine is equal to 1.21% or less. In some embodiments based on the total weight of the composition on a dry matter basis, the amount of arginine is equal to 1.11% or less. In some embodiments based on the total weight of the composition on a dry matter basis, the amount of arginine is equal to less than 1.04%. In some embodiments based on the total weight of the composition on a dry matter basis, the amount of arginine is equal to between 1.04-1.84%. In certain embodiments, compositions may comprise chicken in an amount of 5%, 7.5%, 10%, 12.5%, 15%, 17.5%, 20%, 22.5% or 25% based on the total weight of the composition on a dry matter basis. In certain embodiments, compositions may comprise egg protein in an amount of 4%, 5%, 6%, 7%, 8%, 9%, 10%, 11%, 12%, 13%, 14% or 15% based on the total weight of the composition on a dry matter basis. In certain embodiments, compositions may comprise corn gluten meal in an amount of 6%, 7%, 8%, 9%, 10%, 11%, 12%, 13%, 14%, 15%, 16%, 17%, 18%, 19%, or 20% based on the total weight of the composition on a dry matter basis. In certain embodiments, compositions may comprise a vegetable source in an amount of 0.5%, 0.6%, 0.7%, 0.8%, 0.9%, 1.0%, 1.1%, 1.2%, 1.3%, 1.4%, 1.5%, 1.6%, 1.7%, 1.8%, or 1.9%, or 2.0% based on the total weight of the composition on a dry matter basis. In certain embodiments, compositions may comprise, in addition to tomato pomace, an additional fruit source in an amount of 0.1%, 0.2%, 0.3%, 0.4%, 0.5%, 0.6%, 0.7%, 0.8%, 0.9%, 1.0%, 1.1%, 1.2%, 1.3%, 1.4%, or 1.5% based on the total weight of the composition on a dry matter basis. In certain embodiments, compositions may comprise a carbohydrate selected from millet, brewers rice, oat groats, and combinations thereof in an amount of 5%, 10%, 15%, 20%, 25%, 30%, 35%, 40%, 45%, or 50% based on the total weight of the composition on a dry matter basis. In particular aspects of these embodiments, composition of the invention may comprise a dry weight of a carbohydrate source within a range defined by any two of these values as endpoints.

### Example 7

Table 6 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 6**

| | |
|---|---|
| Protein | from about 5% to about 70%, or from about 10% to about 70%, or from about 10% to about 60% |
| Carbohydrate (preferably a nitrogen-free or essentially nitrogen-free extract) | from about 0% to about 50%, or from about 5% to about 45% |
| Fat | from about 2% to about 50%, or from about 5% to about 50%, or from about 5% to about 40% |
| Dietary fiber | from about 0% to about 40%, or from about 1% to about 20%, or from about 1% to about 5.5% |
| Nutritional balancing agents (e.g., vitamins, and minerals) | from about 0% to about 15%, or from about 2% to about 8% |

### Example 8

Table 7 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 7**

| Description | Content Range (Approx. w/w %) |
|---|---|
| Chicken, livers, hydrolyzed, dry | 25-45 |
| Hyvital^{®} wheat glutamine PN | 0.25-2 |
| Lysine, 1, hydrochloride | 0.1-0.75 |
| Methionine, dl | <0.08 |
| Taurine | 0.075-0.2 |
| Captex^{®} 355 Medium | 1-5 |
| Chained Triglyceride | |
| Cellulose, coarse | 1-5 |
| Beet, pulp | 1-3 |
| OatWell^{®} 22 oat bran | 2-5 |
| Pecan Fiber | 1-5 |
| MEG-3^{®} 0355TG Fish Oil | 0.5-2.5 |
| Ginger Root Powder | 0.5-2 |
| Cranberry Pomace | 0.1-0.4 |
| Pomegranate Extract WS | 0.1-0.4 |
| Green Tea PE 50% EGCG WS | 0.1-0.4 |
| Boswellia PE 65% Boswellic Acids | 0.05-0.3 |
| Sensimune^{™} 75 (Yeast Cell Wall) | 0.05-0.3 |

### Example 9

Table 8 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 8**

| Ingredient | Approx. w/w % |
|---|---|
| Chicken, livers, hydrolyzed, dry | up to 36.79 |
| Corn, starch, common canning | up to 32.45 |
| Choice White Grease | 1.00 |
| Mineral, premix, 2305 | 0.08 |
| Vitamin E, oil, 29% | 0.10 |
| Hyvital ^{®} Wheat Glutamine PN | 1.00 |
| Lysine, 1, hydrochloride | 0.50 |
| Methionine, dl | 0.07 |
| Taurine | 0.10 |
| Captex ^{®} 355 Medium Chained Triglyceride | up to 4.00 |
| Cellulose, coarse | up to 3.00 |
| Lactic acid, food grade | 1.50 |
| Dicalcium phosphate | 1.20 |
| Chicken, liver, digest, optimizer LDPE H | 2.00 |
| Sodium chloride, iodized | 0.40 |
| Choline chloride, liquid, 70% | 0.25 |
| Calcium carbonate | 2.00 |
| Potassium chloride | 0.70 |
| Beet, pulp | up to 2.50 |
| OatWell ^{®} 22 oat bran | up to 3.00 |
| Pecan Fiber | up to 2.00 |
| MEG-3 ^{®} 0355TG Fish Oil | 1.50 |
| Ginger Root Powder | 1.00 |
| Palatant | 0.75 |
| Natural flavor, Pork, Liver, Digest, D'T | 0.50 |
| Glyceryl monostearate | 0.25 |
| Cranberry Pomace | 0.20 |
| Pomegranate Extract WS | 0.20 |
| Green Tea PE 50% EGCG WS | 0.20 |
| Boswellia PE 65% Boswellic Acids | 0.20 |
| Sensimune ^{™} 75 (Yeast Cell Wall) | 0.15 |

### Example 10

Table 9 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 9**

| Ingredient | Approx. w/w % |
|---|---|
| Rice, brewers | up to 25.00 |
| Pea, protein concentrate | 10.00 |
| Chicken Dried 10% Ash | 8.00 |
| Chicken, ground, fresh | 7.00 |
| Sorghum, whole | 6.36 |
| Chicken Meal | 6.14 |
| Pork Fat, Choice White Grease | 1.00 |
| Flax, seed, whole | 3.00 |
| Eggs, dried, granulated | 5.50 |
| Pecan Fiber | 4.80 |
| G03 Buckwheat Groats | 4.00 |
| Oat, groats | 4.00 |
| Captex 355 Medium Chained Triglyceride | 3.00 |
| Chicken, liver, digest, optimizer LDPE H | 2.00 |
| Oat, fiber | 1.50 |
| Beet, pulp, ground, fine | 1.50 |
| Lactic acid, food grade | 1.50 |
| Fish oil, TG, 18/12, NP | 1.20 |
| Flav Gen#1 + CWG | 1.00 |
| Potassium chloride | 0.30 |
| Carnitine, 1, 10% | 0.27 |
| Natural flavor, Pork, Liver, Digest, D'T | 0.25 |
| Choline chloride, liquid, 70% | 0.18 |
| Sensimune 75 (Yeast Cell Wall) | 0.15 |
| Vitamin E, oil, 29% | 0.14 |
| Taurine | 0.10 |
| Sodium chloride, iodized | 0.10 |
| Lysine, 1, hydrochloride | 0.10 |
| Mineral, premix, 2305 | 0.04 |
| Oat Fiber, Fruit, Vegetable blend | 0.04 |
| Dicalcium phosphate | 0.04 |

### Example 11

Table 10 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 10**

| Ingredient | Approx. w/w % |
|---|---|
| Rice, Brewers | - |
| Chicken Meal | 7.00 |
| Pea, protein concentrate | 8.00 |
| Cellulose, coarse | 4.00 |
| Chicken Dried 10% Ash | 6.00 |
| Barley, pearled, cracked | 20.00 |
| Chicken, ground, fresh | 8.00 |
| Flax, seed, whole | 2.00 |
| Coconut oil preserved | 4.00 |
| Chicken, liver, digest, optimizer | 3.00 |

| LDPE H | |
|---|---|
| Lactic acid | 1.50 |
| Methionine, dl | 0.64 |
| Potassium chloride | 0.50 |
| Sodium chloride, iodized | 0.60 |
| Fish oil, TG, 18/12, NP | 0.50 |
| Calcium carbonate | 0.30 |
| Choline chloride, liquid, 70% | 0.25 |
| Carnitine, 1, 10% | 0.30 |
| Vitamin E, oil, 29% | 0.17 |
| Mineral, premix, 2305 | 0.08 |
| Taurine | 0.06 |
| Oat, groats | 10.00 |
| Buckwheat Groats | 6.92 |
| Pea, bran, meal | 5.00 |
| Tomato, pomace, | 5.00 |
| Beet, pulp, ground, fine | 3.00 |

### Example 12

Table 11 describes certain embodiments having proportion of the composition (% of dry weight of component composition) that includes total arginine present at 1.84% or less, and in some embodiments, total arginine present at 1.21% or less, and in some embodiments, total arginine present at 1. 11% or less, and in some embodiments, total arginine present at less than 1.04%, and in some embodiments, total arginine present at 1.04-1.84%,

**TABLE 11**

| Ingredient | Approx. w/w % | Approx. w/w % |
|---|---|---|
| Corn starch | 31.10 | 48.11 |
| Hydrolyzed chicken liver and heart | 37.00 | 32.00 |
| Soybean oil, crude, degummed | 3.60 | 4.66 |
| Cellulose, pelleted | - | 3.94 |
| Chicken, liver, digest, optimizer LDPE H | 2.00 | 2.00 |
| Lactic acid, food grade | 1.50 | 1.50 |
| Calcium carbonate | 1.22 | 1.22 |
| Dicalcium phosphate | 1.22 | 1.22 |
| Choice White Grease/Phos Acid | 1.25 | 1.00 |
| Flav Gen#1 + CWG | 1.25 | 0.75 |
| Glyceryl monostearate | 0.74 | 0.74 |
| Potassium chloride | 0.69 | 0.69 |
| Natural flavor, Pork, Liver, Digest, D'T | 0.75 | 0.50 |
| Sodium chloride, iodized | 0.44 | 0.44 |
| Choline chloride, liquid, 70% | 0.38 | 0.38 |
| Methionine, dl | 0.30 | 0.30 |
| Sodium tripolyphosphate | 0.15 | 0.15 |
| Vitamin premix | 0.12 | 0.12 |
| Mineral, premix, 2305 | 0.07 | 0.07 |
| Taurine | 0.02 | 0.02 |
| Pecan shells, ground | up to 7.00 | - |
| Flax seed whole brown | up to 3.00 | - |
| Beet pulp, ground, fine | up to 2.50 | - |
| Cranberry pomace | up to 1.00 | - |

### Example 13

Table 12 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 12**

| Ingredient | Approx. w/w % | Approx. w/w % |
|---|---|---|
| Chicken meal | 15.36 | 15.36 |
| Rice, brewers | 8.64 | 8.64 |
| Eggs, dried, granulated | 8.00 | 8.00 |
| Corn, gluten, meal | 7.62 | 7.62 |
| Sorghum, whole | 5.00 | 5.00 |
| Choice white grease/Phos Acid | 4.00 | 4.00 |
| Palatant, 12 L, Liquid | 3.00 | 3.00 |
| Lactic acid, food grade | 1.50 | 1.50 |
| Soybean oil, crude, degummed | 1.05 | 1.05 |
| Palatant, ITE2, Dry | 1.00 | 1.00 |
| Potassium chloride | 0.89 | 0.89 |
| Sodium chloride, iodized | 0.61 | 0.61 |
| Calcium carbonate | 0.41 | 0.41 |
| Dicalcium phosphate | 0.25 | 0.25 |
| Vitamin E, oil, 29% | 0.17 | 0.17 |
| Choline chloride, liquid, 70% | 0.16 | 0.16 |
| Mineral, premix, 2305 | 0.06 | 0.06 |
| Tryptophan | 0.04 | 0.04 |
| Taurine | 0.04 | 0.04 |
| Cellulose, pelleted | - | 1.50 |
| Corn, yellow, whole | 26.00 | 40.00 |
| Pecan shells, ground | 7.00 | - |
| Flax seed whole brown | 3.00 | - |
| Beet pulp, ground, fine | 2.50 | 0.50 |
| Cranberry pomace | 1.00 | - |

### Example 14

Table 13 describes certain embodiments having proportion of the composition (% of dry weight of component composition) that includes total arginine present at 1.84% or less, and in some embodiments, total arginine present at 1.21% or less, and in some embodiments, total arginine present at 1.11% or less, and in some embodiments, total arginine present at less than 1.04%, and in some embodiments, total arginine present at 1.04-1.84%,

**TABLE 13**

| Ingredient | Approx. w/w % | Approx. w/w % | Approx. w/w % | Approx. w/w % |
|---|---|---|---|---|
| Protein | 19.7-21.7 | 24.7-26.7 | 24.8-26.8 | 24.8-26.8 |
| Fat | 20.6-22.6 | 16.9-18.9 | 22.0-24.0 | 22.0-24.00 |
| Carbohydrate | 53.8-55.8 | 51.0-53.0 | 46.3-48.3 | 27.6-29.6 |
| Crude Fiber | 0.37-2.37 | 2.6-4.6 | 1.4-3.4 | 21.0-23.0 |

### Example 15

Table 14 shows the % arginine of various pet food ingredients. Those skilled in the art could readily formulate a suitable cat food to provide the daily nutritional requirements for a cat as disclosed in Official Publication of the Association of American Feed Control Officials, Inc. (AAFCO), Atlanta, Ga., (2012). Using the table in "Nutrient Requirements of Domestic Animals: Nutrient Requirements of cats, Revised Edition, National Academy Press Washington. DC. 1986. page 63-67" which is designated Table 10 in the reference and excerpted in relevant part in Table 14 herein, those skilled in the art could readily formulate a suitable cat food to provide the daily nutritional requirements while restricting the level of arginine to between 1.04-1.84% of the total daily nutritional intake based on percent of total dry weight, and in some embodiments arginine at a level of 1.21% or less of the total daily nutritional intake based on percent of total dry weight, and in some embodiments arginine at a level of 1.11% or less of the total daily nutritional intake based on percent of total dry weight

**TABLE 14**

| COMMON NAME *Genus species* | | Dry Matter (%) | Crude Protein (%) | Arginine (%) |
|---|---|---|---|---|
| | Short name | | | |
| BARLEY *Hordeum vulgare* | | | | |
| | grain, | 89 | 13.0 | 0.66 |
| | grain, Pacific Coast | 89 | 10.1 | 0.54 |
| BLOOD, ANIMAL | | | | |
| | fresh | - | - | - |
| | meal spray dehydrated | 93 | 95.6 | 4.10 |
| BONE, ANIMAL | | | | |
| | meal steamed | 90 | 11.4 | 1.79 |
| | phosphate | 98 | - | - |
| BREAD *Triticum aestivum* | | | | |
| | dehydrated (wheat) | 95 | 13.0 | - |
| BUTTERMILK *Bos taurus* | | | | |
| | dehydrated | 92 | 34.4 | 1.17 |
| CANOLA *Brassica napus-Brassica campestris* | | | | |
| | seeds, meal prepressed, solv extd, low erucic acid, low glucosinolates | 93 | 40.9 | 2.49 |
| CASEIN | | | | |
| | dehydrated | 93 | 93 8 | 3.88 |
| CATTLE *Bos taurus* | | | | |
| | chucks | 39 | 47.7 | - |
| | lips, fresh | 30 | 80.0 | - |
| | livers, fresh | 28 | 69.6 | 3.56 |
| | lung, fresh | 24 | 65.0 | 3.10 |
| | spleens, fresh | 24 | 88 7 | - |
| | tripe, dressed (lime treated) | 33 | 46.1 | 5.50 |
| | udders, fresh | 20 | 58.6 | - |
| CORN, DENT YELLOW *Zea mays indentata* | | | | |
| | distillers grains w solubles dehydrated | 93 | 29.3 | 1.05 |
| | distillers solubles, dehydrated | 92 | 31.0 | 1.14 |
| | germ, meal wet milled solv extd | 92 | 22.6 | 1.43 |
| | gluten, meal 60% | 90 | 88 9 | 2.14 |
| | grain, flaked | 90 | 11.2 | 0.49 |
| | hominy feed | 90 | 11.1 | 0.52 |
| | grain | 89 | 9.9 | 0.50 |
| COTTON *Gossypium* spp | | | | |
| seeds, meal prepressed solv extd, 41% protein | | 90 | 46.0 | 5.10 |
| | seeds, meal solv extd, 41% protein | 91 | 45.4 | 4.66 |
| seeds wo/ hulls, meal prepressed solv extd, 50% protein | | 93 | 54 0 | 5.20 |
| CRAB *Callinecles sapidus-Cancer* spp*-Paralithodes camschiatica* | | | | |
| | cannery residue, meal | 92 | 35.0 | 1.80 |
| FISH, ALEWIFE *Pomotobus pseudoharengus* | | | | |
| | meal mech extd | 90 | 69.4 | 4.96 |
| | whole, fresh | 26 | 75.8 | - |
| FISH, ANCHOVY *Engraulis ringen* | | | | |
| | meal mech extd | 92 | 69.8 | 3.98 |
| FISH, CARP *Cyprinus carpio* | | | | |
| | meal mech extd | 90 | 58.6 | - |
| | whole, fresh | 31 | 61.9 | - |
| FISH, CATFISH *Ictalurus* spp | | | | |
| | meal, mech extd | 94 | 55.3 | - |
| | whole, fresh | 22 | - | - |
| FISH, COD *Gadus morrhua-Gadus macrocephalus* | | | | |
| | meal, mech extd | 84 | 73.1 | - |
| | whole, fresh | - | - | - |
| FISH, FLOUNDER Bothidae (family)-Pleuronectidae (family) | | | | |
| | whole, fresh | 17 | 88.2 | - |
| FISH, HADDOCK *Melanogrammus aeglefinus* | | | | |
| | whole, fresh | 10 | 93.8 | - |
| FISH, HAKE *Merluccius* spp-*Urophycis* spp | | | | |
| | whole, boiled | 26 | 57.8 | - |
| | whole, boiled acidified | 25 | - | - |
| | whole, fresh | 20 | 50.6 | - |
| FISH, HERRING *Clupea harengus* | | | | |
| | meal, mech extd | 93 | 77.7 | 5.20 |
| | whole, fresh | 29 | 63.1 | - |
| FISH, MACKEREL *Scomber scrombus* | | | | |
| | Atlantic, whole, fresh | 28 | 48.7 | - |
| FISH, MACKEREL *Scomber japonicus* | | | | |
| | Pacific, whole, fresh | 30 | 72.5 | - |
| FISH, MENHADEN *Brevoartia tyrannus* | | | | |
| | meal, mech extd | 92 | 65.8 | 4.12 |
| FISH, REDFISH *Sciaenops occellata* | | | | |
| | meal, mech extd | 93 | 61.0 | 4.36 |
| FISH, ROCKFISH *Sebastodes* spp | | | | |
| | whole, fresh | 21 | 89.6 | - |
| FISH, SALMON *Oncorhynchus* spp*-Salmo* spp | | | | |
| | meal, mech extd | 94 | 65.3 | 5.50 |
| | whole, fresh | 32 | 66.2 | - |
| FISH, SARDINE *Clupea* spp*-Sardinops* spp *Osmerus* spp | | | | |
| | meal, mech extd | 93 | 70.0 | 2.93 |
| FISH, SMELT Soleidae (family) | | | | |
| | whole, fresh | 21 | 89.6 | - |
| FISH, TUNA *Thunnus thynnus-Thunnus albacares* | | | | |
| | cannery residue | 81 | 55.5 | 3.65 |
| | meal, mech extd | 93 | 63.6 | 3.44 |
| FISH, TURBOT *Psetta maxima* | | | | |
| | whole, fresh | 25 | 57.3 | - |
| FISH, WHITE Gadidae (family)-Lophidae (family)-Rajidae (family) | | | | |
| | meal, mech extd | 93 | 68.4 | 4.42 |
| FISH, WHITING *Gadus merlangus* | | | | |
| | whole, fresh | 23 | 69.9 | - |
| FISH | | | | |
| | livers, mech extd | 93 | 67.7 | - |
| | racks, dehydrated grad (bones w heads) | - | - | |
| | solubles, condensed | 51 | 61.8 | - |
| | solubles, dehydrated | 92 | 69.1 | - |
| FLAX *Linum usiatissimum* | | | | |
| | seeds, meal solv extd (linseed meal) | 90 | 38.4 | 3.25 |
| HAMBURGER *Bos taurus* | | | | |
| | fresh, 10% fat | 38 | 65.3 | 2.21 |
| | fresh, 20% fat | 39 | 45.0 | - |
| HORSE *Equus caballus* | | | | |
| | meat, fresh | 31 | 63.6 | - |
| | meal w bone fresh | 36 | 51.4 | - |
| LIVER, ANIMAL | | | | |
| | meat, dehydrated | 92 | 71.3 | 4.50 |
| MEAT, ANIMAL | | | | |
| | meal, rendered | 92 | 50.1 | 4.05 |
| | w blood, w bone, meal tankage rendered | 93 | 50.2 | 3.03 |
| | w bone, meal rendered | 93 | 53.9 | 3.80 |
| MILK | | | | |
| | dehydrated (cattle) | 96 | 26.6 | 0.96 |
| | fresh (cattle) | 12 | 26.7 | - |
| | skimmed dehydrated (cattle) | 94 | 35.4 | - |
| | skimmed fresh (cattle) | 10 | 31.2 | - |
| | cottage cheese | 21 | 81.0 | - |
| | whey albumin (cattle) | 92 | 52.5 | - |
| MILLET, FOXTAIL *Setaria italica* | | | | |
| | grain | 89 | 12.8 | 0.72 |
| MOLASSES | | | | |
| | beet sugar, molasses, mt 48% | 78 | 8.5 | - |
| invert sugar mt 79.5 degrees brix, sugar cane, molasses dehydrated | | 94 | 10.3 | - |
| sugar cane, molasses dehydrated, mt 46% invert, sugar mt 79.5 degrees brix | | 74 | 5.8 | - |
| OATS *Avena sativa* | | | | |
| cereal by-product, lt 4% fiber (feeding oat meal) (oat middlings) | | 91.0 | 16.3 | 0.89 |
| | grain | 89 | 12.8 | 0.90 |
| | groats | 90 | 17.6 | 0.99 |
| | hulls | 92 | 5.2 | 0.16 |
| PEA *Pisum* spp | | | | |
| | seeds | 90 | 26.4 | 1.56 |
| PEANUT *Arachis hypogaca* | | | | |
| | kernels, meal solv extd (peanut meal) | 93 | 54.5 | 5.91 |
| POTATO *Solanum tuberosum* | | | | |
| | tubers, dehydrated | 91 | 8.9 | 0.28 |
| POULTRY *Gallus domesticus* | | | | |
| | heads, fresh | 33 | 57.6 | - |
| | broilers, whole fresh | - | - | - |
| | eggs, fresh whole | 30 | 42.6 | 2.90 |
| | eggs, fresh white | 13 | 87.1 | 4.96 |
| | feet fresh | 33 | 54.5 | - |
| | gizzards fresh | 25 | 80.4 | - |
| | by-products, fresh (viscera with feet and heads) | - | - | - |
| | by-product, meal rendered (viscera with feet and heads) | 93 | 62.4 | 4.30 |
| | hens, whole fresh | - | - | - |
| | chicken viscera | 26 | 54.4 | - |
| POULTRY FEATHERS | | | | |
| | hydrolyzed meal | 93 | 92.9 | 5.83 |
| RICE *Orzya sativa* | | | | |
| | bran w germ (rice bran) | 91 | 14.2 | 0.98 |
| | grain | 89 | 8.6 | 0.65 |
| | groats, polished | 89 | 8.2 | 0.50 |
| | polishings | 90 | 13.6 | 0.87 |
| RYE *Secate cereale* | | | | |
| | grain | 88 | 13.7 | 0.60 |
| SEAWEED, KELP | | | | |
| | Laminariales order, Fucales order | 91 | 7.1 | - |
| SESAME *Sesamum indicum* | | | | |
| | seeds, meal mech extd | 93 | 47.1 | 5.30 |
| SHRIMP *Pandalus* spp*-Panacus* spp | | | | |
| | cannery residual meal (shrimp meal) | 90 | 43.0 | 2.58 |
| SORGHUM *Sorghum vulgare* | | | | |
| | grain | 90 | 10.0 | 0.40 |
| SOYBEAN *Glycine max* | | | | |
| | seeds, meal mech extd | 89 | 49.4 | 3.69 |
| | seeds wo hulls, meal mech extd | 90 | 53.9 | 3.80 |
| | flour, concentrate 70% protein | 93 | 90.4 | 7.20 |
| | flour, oil residue solvent extd | 93 | 55.3 | 4.58 |
| | isolate 90% protein | 96 | 94.0 | 3.80 |
| | seeds, heat processed | 90 | 41.1 | 3.11 |
| SUCROSE | | | | |
| | sucrose | 99 | - | - |
| SUNFLOWER *Helianthus* spp. | | | | |
| | seeds wo hulls, meal mech extd | 93 | 43.7 | 3.66 |
| | seeds wo hulls, meal solv extd | 93 | 43.8 | 3.76 |
| SWINE *Sus scrofa* | | | | |
| | livers fresh | 30.5 | 69.8 | 3.69 |
| | lungs fresh | - | - | - |
| TOMATO *Lycopersicon esculentum* | | | | |
| | pomace dehydrated | 92 | 23.0 | 1.30 |
| TURKEY *Meleagris gallopavo* | | | | |
| | mature birds, offal fresh | - | - | - |
| | young heads, offal fresh | - | - | - |
| | viscera fresh | 31 | 43.4 | - |
| WHALE *balaena glacialis-Balaenoptera* spp.-*Physter catadon* | | | | |
| | meat fresh | 29 | 70.8 | - |
| WHEAT *Triticum aestivum* | | | | |
| | bran | 90 | 17.4 | 1.0 |
| | flour, hard red spring | 89 | 14.4 | 0.55 |
| | flour, lt 2% fiber | 88 | 15.5 | 0.52 |
| | germ meal | 88 | 27.6 | 1.65 |
| | grain | 89 | 14.7 | 0.66 |
| | grain, hard red spring | 88 | 16.2 | 0.68 |
| | grain, hard red winter | 87 | 16.2 | 0.83 |
| | grain, screenings | 89 | 14.9 | 0.60 |
| | grain, soft red winter | 88 | 12.9 | 0.62 |
| | grain, soft white winter | 89 | 11.5 | 0.55 |
| | grits | 90 | 12.7 | - |
| | mill run, lt 9.5% fiber | 90 | 16.7 | 0.59 |
| | red dog, lt 4.5% fiber | 88 | 17.4 | 0.84 |
| | shorts, lt 7.0% fiber | 88 | 18.7 | 1.08 |
| | middlings, lt 9.5% fiber | 88 | 17.6 | - |
| WHEAT DURUM *Triticum durum* | | | | |
| | grain | 88 | 15.6 | 0.52 |
| WHEY *Bos taurus* | | | | |
| | dehydrated | 93 | 12.9 | 0.32 |
| | low lactose, dehydrated (dried whey product) | 91 | 17.0 | 1.14 |
| YEAST *Saccaromyces cerevisae* | | | | |
| | brewers dehydrated | 93 | 47.7 | 2.24 |
| | primary dehydrated | 93 | 51.8 | - |
| YEAST *Candida utilis* | | | | |
| | petroleum solv extd dehydrated | 92 | 51.1 | - |
| YEAST *Torulopsis utilis* | | | | |
| | torula dehydrated | 93 | 50.8 | 2.80 |

### Example 16

Raw data is show in in Tables 15 and 16 below from the genome wide association analysis described above which showed that there was a relationship between the AGXT2 gene and circulating concentrations of beta-aminoisobutyrate and 2-oxoarginine, which is the product of the transamination of arginine.

Raw data is show in in Tables 17 and 18 below from experiments comparing circulating concentrations of beta-aminoisobutyrate in animals fed different diets.

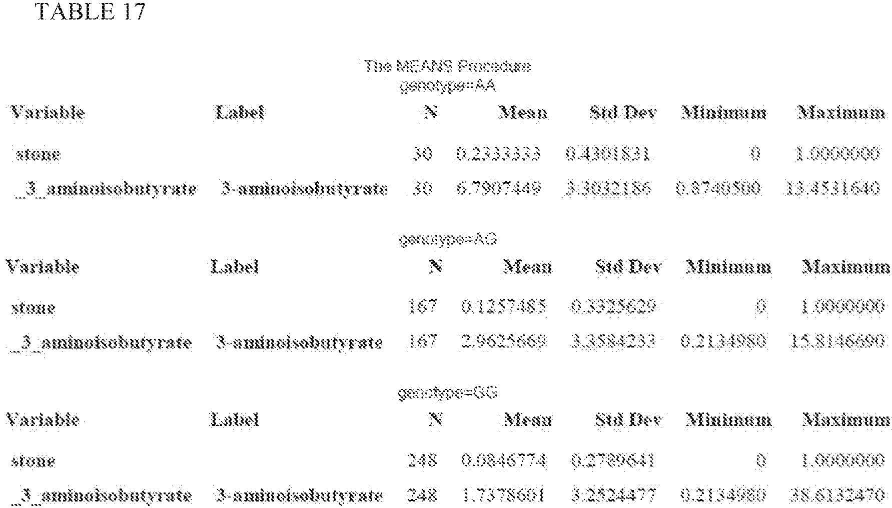

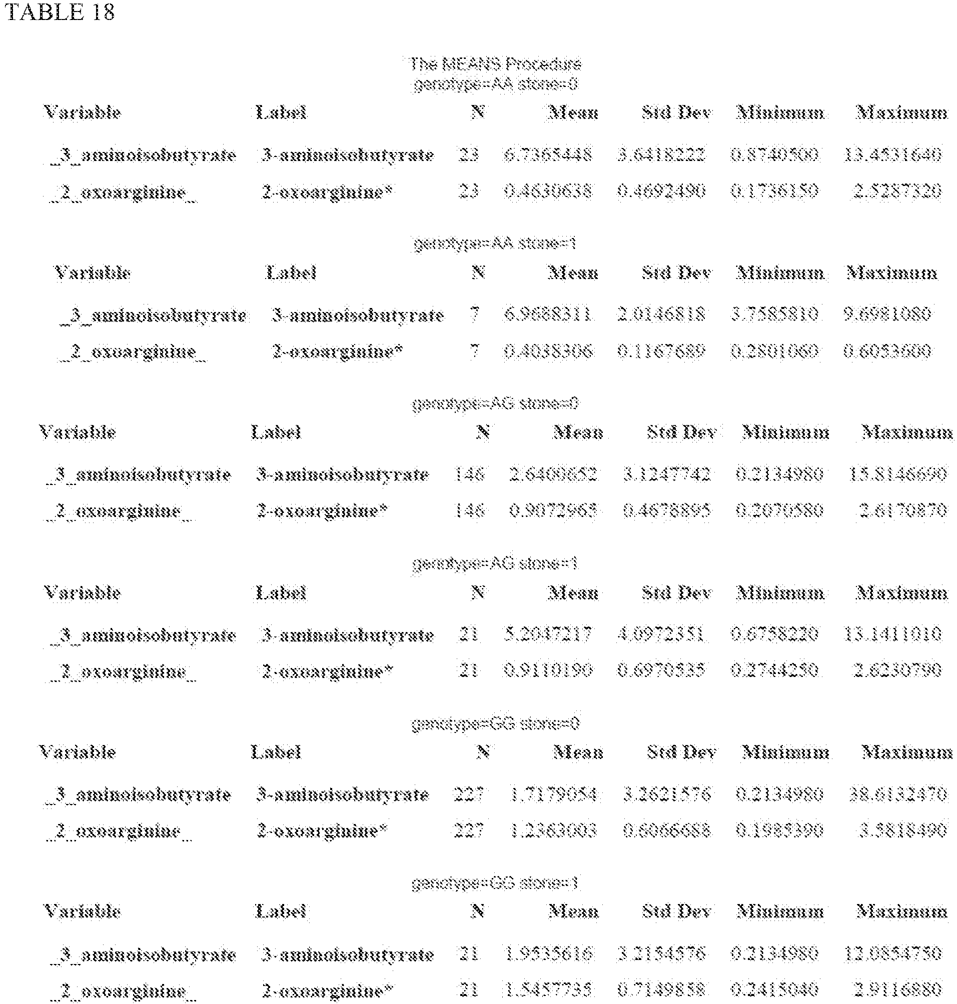

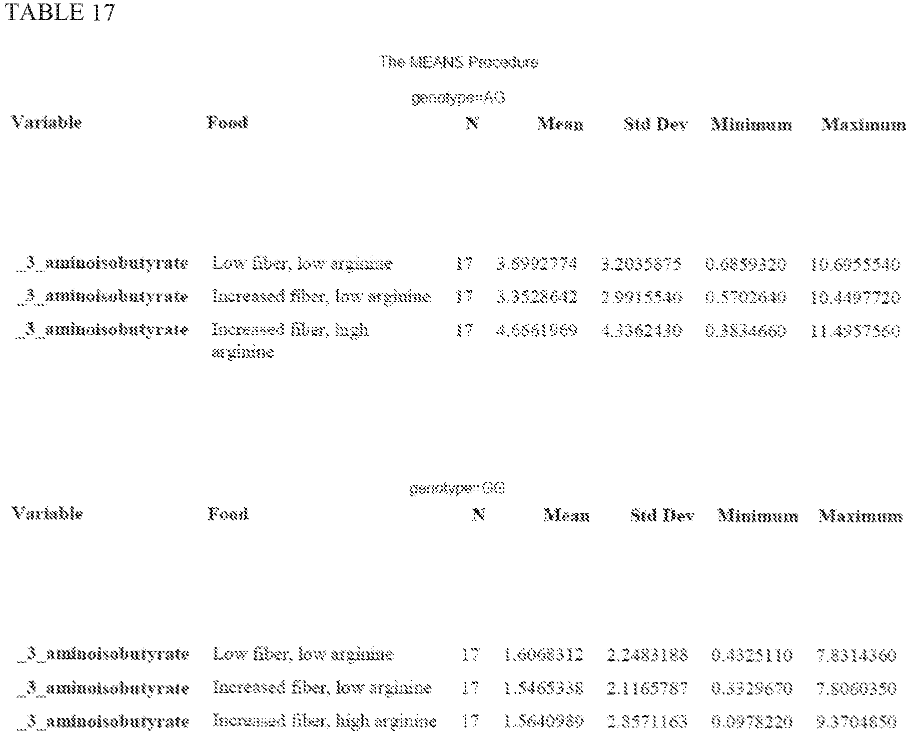

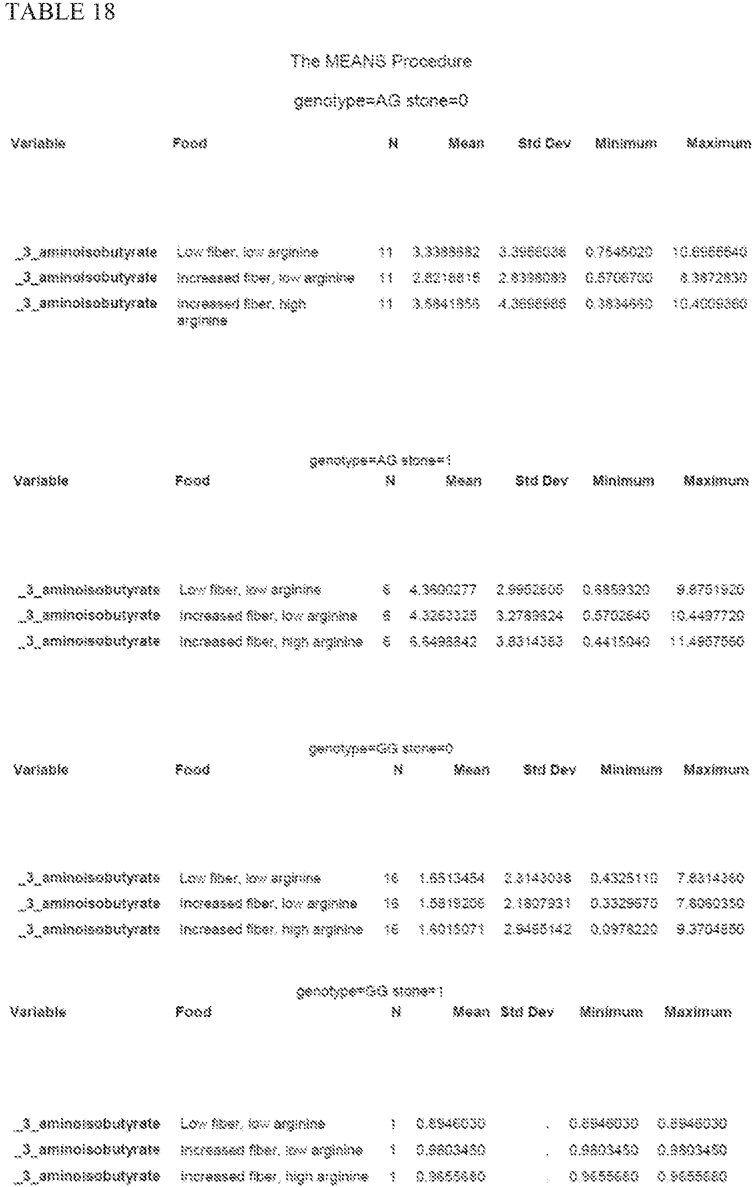

## Claims

1. A method of identifying a feline subject with an increased likelihood of developing calcium oxalate stones, the method comprising:
analyzing a biological sample obtained from the feline subject for the presence of one or two copies of minor allele A of SNP A1_212891692, felCat8; and
wherein the presence of one or two copies of minor allele A of SNP A1_212891692 indicates that the feline subject has an increased likelihood of developing calcium oxalate stones.

2. The method of claim 1, wherein the sample is a genomic DNA sample, optionally, wherein the sample is obtained from blood, saliva, follicle root, nasal swab or oral swab of the feline subject, preferably saliva.

3. The method of any one of claims 1-2, wherein the sample is analyzed by performing at least one nucleic acid analysis technique selected from: DNA sequencing, restriction enzyme digest, polymerase chain reaction (PCR), hybridization, real-time PCR, reverse transcriptase PCR, or ligase chain reaction.

4. The method of any one of claims 1-2, wherein the sample is analyzed by performing at least one nucleic acid analysis technique selected from: analysis using a whole genome SNP chip, single-stranded conformational polymorphism (SSCP) assay, restriction fragment length polymorphism (RFLP), automated fluorescent sequencing; clamped denaturing gel electrophoresis (CDGE); denaturing gradient gel electrophoresis (DGGE), mobility shift analysis, restriction enzyme analysis, heteroduplex analysis, chemical mismatch cleavage (CMC), RNase protection assays, use of polypeptides that recognize nucleotide mismatches, allele-specific PCR, sequence analysis, and SNP genotyping.

5. The method of any one of claims 1-2, wherein the sample is analyzed by performing at least one nucleic acid analysis technique selected from: hybridization-based methods, enzyme-based methods, post-amplification methods based on physical properties of DNA, and sequencing methods.

6. The method of any one of claims 1-2, wherein the sample is analyzed by performing at least one nucleic acid analysis technique selected from: hybridization-based methods selected from the group consisting of dynamic allele-specific hybridization, molecular beacon methods and SNP microarrays; enzyme-based methods selected from the group consisting of restriction fragment length polymorphism (RFLP), PCR-based methods, Flap endonuclease, primer extension methods, 5'- nuclease and oligonucleotide ligation assay; post-amplification methods based on physical properties of DNA selected from the group consisting of single strand conformation polymorphism, temperature gradient gel electrophoresis, denaturing high performance liquid chromatography, high-resolution amplicon melting, DNA mismatch-binding proteins, SNPlex, and surveyor nuclease assay; and sequencing methods

7. The method of any one of the foregoing claims, further comprising:
analyzing a biological sample obtained from the feline subject to determine the feline subject's beta-amino isobutyrate concentration; and
comparing the feline subject's beta-amino isobutyrate concentration with a positive reference beta-amino isobutyrate concentration value that is representative of beta-amino isobutyrate concentration of a cat with an increased likelihood of developing calcium oxalate stones, wherein the feline subject's beta-amino isobutyrate concentration being equal to or greater than the positive reference beta-amino isobutyrate concentration value indicates that the feline subject has an increased likelihood of developing calcium oxalate stones.

8. The method of any one of claims 1-6 further comprising
analyzing a biological sample obtained from the feline subject to determine the feline subject's beta-amino isobutyrate concentration;
analyzing a positive control sample to determine the positive control sample's beta-amino isobutyrate concentration, wherein the positive control sample contains beta-amino isobutyrate in a concentration representative of a cat with an increased likelihood of developing calcium oxalate stones; and
comparing the feline subject's beta-amino isobutyrate concentration with the positive control sample beta-amino isobutyrate concentration, wherein the feline subject's beta-amino isobutyrate concentration being equal to or greater than the positive control sample beta-amino isobutyrate concentration indicates that the feline subject has an increased likelihood of developing calcium oxalate stones.

9. The method of any one of claims 1-8 further comprising:
analyzing a biological sample obtained from the feline subject to determine the feline subject's 2-oxoarginine concentration; and
comparing the feline subject's 2-oxoarginine concentration with a positive reference 2-oxoarginine concentration value that is representative of 2-oxoarginine concentration of a cat with an increased likelihood of developing calcium oxalate stones, wherein the feline subject's 2-oxoarginine concentration being equal to or less than the positive reference 2-oxoarginine concentration value indicates that the feline subject has an increased likelihood of developing calcium oxalate stones.

10. The method of any one of claims 1-8 further comprising:
analyzing a biological sample obtained from the feline subject to determine the feline subject's 2-oxoarginine concentration;
analyzing a positive control sample to determine the positive control sample's 2-oxoarginine concentration, wherein the positive control sample contains 2-oxoarginine in a concentration representative of a cat with an increased likelihood of developing calcium oxalate stones; and
comparing the feline subject's 2-oxoarginine concentration with the positive control sample 2-oxoarginine concentration, wherein the feline subject's 2-oxoarginine concentration being equal to or less than the positive control sample 2-oxoarginine concentration indicates that the feline subject has an increased likelihood of developing calcium oxalate stones.

11. A method of identifying a feline subject that has an increased likelihood of developing calcium oxalate stones, the method comprising:
analyzing a biological sample obtained from the feline subject to determine the feline subject's 2-oxoarginine concentration; and
comparing the feline subject's 2-oxoarginine concentration with a positive reference 2-oxoarginine concentration value that is representative of 2-oxoarginine concentration of a cat with an increased likelihood of developing calcium oxalate stones, wherein the feline subject's 2-oxoarginine concentration being equal to or less than the positive reference 2-oxoarginine concentration value indicates that the feline subject has an increased likelihood of developing calcium oxalate stones.

12. A method of identifying a feline subject that has an increased likelihood of developing calcium oxalate stones, the method comprising:
analyzing a biological sample obtained from the feline subject to determine the feline subject's 2-oxoarginine concentration;
analyzing a positive control sample to determine the positive control sample's 2-oxoarginine concentration, wherein the positive control sample contains 2-oxoarginine in a concentration representative of a cat with an increased likelihood of developing calcium oxalate stones; and
comparing the feline subject's 2-oxoarginine concentration with the positive control sample 2-oxoarginine concentration, wherein the feline subject's 2-oxoarginine concentration being equal to or less than the positive control sample 2-oxoarginine concentration indicates that the feline subject has an increased likelihood of developing calcium oxalate stones.

13. A low arginine nutritional composition for use in a method of reducing risk of calcium oxalate stone formation in a feline subject, the method comprising:
identifying the feline subject as being a feline subject with an increased likelihood of developing calcium oxalate stones according to any of claims 1-12; and
feeding the feline subject a daily diet of a low arginine nutritional composition, wherein the daily diet of the low arginine nutritional composition contains 1.84% or less of arginine as the percentage of dry matter daily intake.

14. The low arginine nutritional composition for use according to claim 13 wherein the daily diet of the low arginine nutritional composition contains 1.21% or less of arginine as the percentage of dry matter daily intake, in particular 1.11% or less of arginine as the percentage of dry matter daily intake, more particularly contains less than 1.04% arginine as the percentage of dry matter daily intake.

15. The low arginine nutritional composition for use according to claim 13 wherein the daily diet of the low arginine nutritional composition contains 1.04-1.84% arginine as the percentage of dry matter daily intake

## Patentansprüche

1. Verfahren zur Identifizierung einer Katze mit einer erhöhten Wahrscheinlichkeit Kalziumoxalatsteine zu entwickeln, wobei das Verfahren folgendes umfasst:
Analysieren einer von der Katze erhaltenen biologischen Probe auf das Vorhandensein von einer oder zwei Kopien des Minor-Allels A des SNP A1_212891692; und
wobei das Vorhandensein von einer oder zwei Kopien des Minor-Allels A des SNP A1_212891692 anzeigt, dass die Katze eine erhöhte Wahrscheinlichkeit hat Kalziumoxalatsteine zu entwickeln.

2. Verfahren nach Anspruch 1, wobei die Probe eine genomische DNA-Probe ist, wobei die Probe gegebenenfalls aus Blut, Speichel, Follikelwurzel, Nasenabstrich oder Mundabstrich der Katze, vorzugsweise aus Speichel, gewonnen wird.

3. Verfahren nach einem der Ansprüche 1-2, wobei die Probe durch Durchführung mindestens einer Nukleinsäureanalysetechnik analysiert wird, die ausgewählt ist aus: DNA-Sequenzierung, Restriktionsenzymverdau, Polymerasekettenreaktion (PCR), Hybridisierung, Echtzeit-PCR, Reverse-Transkriptase-PCR oder Ligasekettenreaktion.

4. Verfahren nach einem der Ansprüche 1-3, wobei die Probe analysiert wird, indem mindestens eine Nukleinsäureanalysetechnik durchgeführt wird, die ausgewählt ist aus: Analyse unter Verwendung eines Ganzgenom-SNP-Chips, Einzelstrangkonformationspolymorphismus-Assay (SSCP), Restriktionsfragmentslängen-Polymorphismus (RFLP), automatisierter Fluoreszenzsequenzierung; Denaturierender Clamped-Gelelektrophorese (CDGE); Denaturierende Gradienten-Gelelektrophorese (DGGE), Mobilitätsverschiebungsanalyse, Restriktionsenzymanalyse, Heteroduplex-Analyse, chemischer Mismatch-Spaltung (CMC), RNase-Schutz-Assays, Verwendung von Nukleotid-Fehlpaarungen erkennenden Polypeptiden, allelspezifischer PCR, Sequenzanalyse und SNP-Genotypisierung.

5. Verfahren nach einem der Ansprüche 1-2, wobei die Probe analysiert wird, indem mindestens eine Nukleinsäure-Analysetechnik durchgeführt wird, die ausgewählt ist aus: hybridisierungsbasierten Methoden, enzymbasierten Methoden, auf physikalischen Eigenschaften der DNA basierenden Postamplifikationsmethoden und Sequenzierungsmethoden.

6. Verfahren nach einem der Ansprüche 1-2, wobei die Probe analysiert wird, indem mindestens eine Nukleinsäureanalysetechnik durchgeführt wird, die ausgewählt ist aus: hybridisierungsbasierten Methoden, die ausgewählt sind aus dynamischer allelspezifischer Hybridisierung, molekularen Beacon-Methoden und SNP-Microarrays; enzymbasierten Methoden, die ausgewählt aus Restriktionsfragmentslängenpolymorphismus (RFLP), PCR-basierten Methoden, Flap-Endonuklease, Primer-Extensionsmethoden, 5'-Nuklease-und-Oligonukleotid-Ligationsassay; auf physikalischen Eigenschaften der DNA basierenden Postamplifikationsmethoden, die ausgewählt sind aus Einzelstrangkonformationspolymorphismus, Temperaturgradienten-Gelelektrophorese, Denaturierender Hochleistungs-Flüssigkeitschromatographie, hochauflösender Amplikonschmelzung, DNA-Mismatch-bindenden Proteinen, SNPlex und Surveyor-Nuklease-Assay; und Sequenzierungsmethoden.

7. Verfahren nach einem der vorangehenden Ansprüche, das ferner folgendes umfasst:
Analysieren einer von der Katze erhaltenen biologischen Probe um die Beta-Aminoisobutyratkonzentration der Katze zu bestimmen; und
Vergleichen der Beta-Aminoisobutyratkonzentration der Katze mit einem positiven Referenzwert der Beta-Aminoisobutyratkonzentration, der für die Beta-Aminoisobutyratkonzentration einer Katze mit einer erhöhten Wahrscheinlichkeit Kalziumoxalatsteine zu entwickeln repräsentativ ist, wobei die Beta-Aminoisobutyratkonzentration der Katze, wenn sie gleich oder größer ist als der positive Referenzwert der Beta-Aminoisobutyratkonzentration, anzeigt, dass die Katze eine erhöhte Wahrscheinlichkeit hat Kalziumoxalatsteine zu entwickeln.

8. Verfahren nach einem der Ansprüche 1-6, das ferner folgendes umfasst
das Analysieren einer von der Katze erhaltenen biologischen Probe um die Beta-Aminoisobutyratkonzentration der Katze zu bestimmen;
Analysieren einer Positivkontrollprobe, um die Beta-Aminoisobutyratkonzentration der Positivkontrollprobe zu bestimmen, wobei die Positivkontrollprobe Beta-Aminoisobutyrat in einer Konzentration enthält, die für eine Katze mit einer erhöhten Wahrscheinlichkeit Kalziumoxalatsteine zu entwickeln repräsentativ ist; und
Vergleichen der Beta-Aminoisobutyratkonzentration der Katze mit der Beta-Aminoisobutyratkonzentration der Positivkontrollprobe, wobei die Beta-Aminoisobutyratkonzentration der Katze, wenn sie gleich oder größer als die Beta-Aminoisobutyratkonzentration der Positivkontrollprobe ist, anzeigt, dass die Katze eine erhöhte Wahrscheinlichkeit hat Kalziumoxalatsteine zu entwickeln.

9. Verfahren nach einem der Ansprüche 1-8, das ferner folgendes umfasst:
Analysieren einer von der Katze erhaltenen biologischen Probe um die 2-Oxoargininkonzentration der Katze zu bestimmen; und
Vergleichen der 2-Oxoargininkonzentration der Katzen mit einem positiven Referenzwert der 2-Oxoargininkonzentration, welcher für die 2-Oxoargininkonzentration einer Katze mit einer erhöhten Wahrscheinlichkeit Kalziumoxalatsteine zu entwickeln repräsentativ ist, wobei die 2-Oxoargininkonzentration der Katze, wenn sie gleich oder kleiner als der positive Referenzwert der 2-Oxoargininkonzentration ist, anzeigt, dass die Katze eine erhöhte Wahrscheinlichkeit hat Kalziumoxalatsteine zu entwickeln.

10. Verfahren nach einem der Ansprüche 1-8, das ferner folgendes umfasst:
Analysieren einer von der Katze erhaltenen biologischen Probe um die 2-Oxoargininkonzentration der Katze zu bestimmen;
Analysieren einer Positivkontrollprobe, um die 2-Oxoargininkonzentration der Positivkontrollprobe zu bestimmen, wobei die Positivkontrollprobe 2-Oxoarginin in einer Konzentration enthält, die für eine Katze mit einer erhöhten Wahrscheinlichkeit Kalziumoxalatsteine zu entwickeln repräsentativ ist; und
Vergleichen der 2-Oxoargininkonzentration der Katze mit der 2-Oxoargininkonzentration der Positivkontrollprobe, wobei die 2-Oxoargininkonzentration der Katze, wenn sie gleich oder geringer als die 2-Oxoargininkonzentration der Positivkontrollprobe ist, anzeigt, dass die Katze eine erhöhte Wahrscheinlichkeit hat Kalziumoxalatsteine zu entwickeln.

11. Verfahren zur Identifizierung einer Katze, die eine erhöhte Wahrscheinlichkeit hat Kalziumoxalatsteine zu entwickeln, wobei das Verfahren folgendes umfasst:
Analysieren einer von der Katze erhaltenen biologischen Probe um die 2-Oxoargininkonzentration der Katze zu bestimmen; und
Vergleichen der 2-Oxoargininkonzentration der Katze mit einem positiven Referenzwert der 2-Oxoargininkonzentration, der für die 2-Oxoargininkonzentration einer Katze mit einer erhöhten Wahrscheinlichkeit Kalziumoxalatsteine zu entwickeln repräsentativ ist, wobei die 2-Oxoargininkonzentration der Katze, wenn sie die gleich oder geringer als der positive Referenzwert der 2-Oxoargininkonzentration ist, anzeigt, dass die Katze eine erhöhte Wahrscheinlichkeit hat Kalziumoxalatsteine zu entwickeln.

12. Verfahren zur Identifizierung einer Katze, die eine erhöhte Wahrscheinlichkeit hat Kalziumoxalatsteine zu entwickeln, wobei das Verfahren folgendes umfasst:
Analysieren einer von der Katze erhaltenen biologischen Probe um die 2-Oxoargininkonzentration der Katze zu bestimmen;
Analysieren einer positiven Kontrollprobe, um die 2-Oxoargininkonzentration der Positivkontrollprobe zu bestimmen, wobei die Positivkontrollprobe 2-Oxoarginin in einer Konzentration enthält, die für eine Katze mit einer erhöhten Wahrscheinlichkeit Kalziumoxalatsteine zu entwickeln repräsentativ ist; und
Vergleichen der 2-Oxoargininkonzentration der Katze mit der 2-Oxoargininkonzentration der Positivkontrollprobe, wobei die 2-Oxoargininkonzentration der Katze, wenn sie gleich oder geringer als die 2-Oxoargininkonzentration der Positivkontrollprobe ist, anzeigt, dass die Katze eine erhöhte Wahrscheinlichkeit hat Kalziumoxalatsteine zu entwickeln.

13. Argininarme Futterzusammensetzung zur Verwendung in einem Verfahren zur Verringerung des Risikos der Kalziumoxalatsteinbildung bei einer Katze, wobei das Verfahren folgendes umfasst:
Identifizierung der Katze als Katze mit einer erhöhten Wahrscheinlichkeit Kalziumoxalatsteine zu entwickeln nach einem der Ansprüche 1-12; und
Füttern der Katze mit einer täglichen Diät einer argininarmen Futterzusammensetzung, wobei die tägliche Diät der argininarmen Futterzusammensetzung 1,84% oder weniger Arginin als Prozentsatz der täglichen Trockenmasseaufnahme enthält.

14. Argininarme Futterzusammensetzung zur Verwendung gemäß Anspruch 13, wobei die tägliche Diät der argininarmen Futterzusammensetzung 1,21 % oder weniger Arginin als Prozentsatz der täglichen Trockenmasseaufnahme enthält, insbesondere 1,11 % oder weniger Arginin als Prozentsatz der täglichen Trockenmasseaufnahme, insbesondere weniger als 1,04 % Arginin als Prozentsatz der täglichen Trockenmasseaufnahme.

15. Argininarme Futterzusammensetzung zur Verwendung gemäß Anspruch 13, wobei die tägliche Diät der argininarmen Futterzusammensetzung 1,04-1,84% Arginin als Prozentsatz der täglichen Trockenmasseaufnahme enthält.

## Revendications

1. Procédé d'identification d'un sujet félin présentant une probabilité accrue de développer des calculs d'oxalate de calcium, le procédé comprenant :
l'analyse d'un échantillon biologique obtenu à partir du sujet félin pour la présence d'une ou deux copies de l'allèle mineur A du SNP A1_212891692, felCat8 ; et
dans lequel la présence d'une ou deux copies de l'allèle mineur A du SNP A1_212891692 indique que le sujet félin présente une probabilité accrue de développer des calculs d'oxalate de calcium.

2. Procédé selon la revendication 1, dans lequel l'échantillon est un échantillon d'ADN génomique, éventuellement, dans lequel l'échantillon est obtenu à partir de sang, de salive, de racine de follicule, d'écouvillon nasal ou d'écouvillon buccal du sujet félin, de préférence de salive.

3. Procédé selon l'une quelconque des revendications 1 à 2, dans lequel l'échantillon est analysé en effectuant au moins une technique d'analyse d'acide nucléique choisie parmi : le séquençage de l'ADN, la digestion par des enzymes de restriction, la réaction en chaîne par polymérase (PCR), l'hybridation, la PCR en temps réel, la PCR par transcriptase inverse ou la réaction en chaîne par ligase.

4. Procédé selon l'une quelconque des revendications 1 à 2, dans lequel l'échantillon est analysé en effectuant au moins une technique d'analyse d'acide nucléique choisie parmi : une analyse utilisant une puce SNP du génome entier, un test de polymorphisme de conformation simple brin (SSCP), un polymorphisme de longueur de fragment de restriction (RFLP), un séquençage par fluorescence automatisé ; une électrophorèse sur gel dénaturant clampé (CDGE) ; une électrophorèse sur gel à gradient dénaturant (DGGE), une analyse des changements de mobilité, une analyse par des enzymes de restriction, une analyse d'hétéroduplex, un clivage par mésappariement chimique (CMC), des tests de protection de la RNase, une utilisation de polypeptides qui reconnaissent les mésappariements de nucléotides, une PCR spécifique d'allèles, une analyse de séquence et un génotypage des SNP.

5. Procédé selon l'une quelconque des revendications 1 à 2, dans lequel l'échantillon est analysé en effectuant au moins une technique d'analyse d'acide nucléique choisie parmi : les méthodes basées sur l'hybridation, les méthodes basées sur les enzymes, les méthodes post-amplification basées sur les propriétés physiques de l'ADN, et les méthodes de séquençage.

6. Procédé selon l'une quelconque des revendications 1 à 2, dans lequel l'échantillon est analysé en effectuant au moins une technique d'analyse d'acide nucléique choisie parmi : les méthodes basées sur l'hybridation choisies dans le groupe constitué par l'hybridation dynamique spécifique d'allèle, les méthodes de balises moléculaires et les puces à ADN de SNP ; les méthodes basées sur des enzymes choisies dans le groupe constitué par le polymorphisme de longueur de fragment de restriction (RFLP), les méthodes basées sur la PCR, l'endonucléase Flap, les méthodes d'extension d'amorce, le test de 5'-nucléase et ligature d'oligonucléotides ; les méthodes post-amplification basées sur les propriétés physiques de l'ADN choisies dans le groupe constitué par le polymorphisme de conformation simple brin, l'électrophorèse sur gel à gradient de température, la chromatographie liquide haute performance dénaturante, la fusion d'amplicons à haute résolution, les protéines de liaison aux mésappariements d'ADN, le SNPlex et le test de nucléase Surveyor ; et les méthodes de séquençage.

7. Procédé selon l'une quelconque des revendications précédentes, comprenant en outre :
l'analyse d'un échantillon biologique obtenu à partir du sujet félin pour déterminer la concentration en bêta-amino isobutyrate du sujet félin ; et
la comparaison de la concentration en bêta-amino isobutyrate du sujet félin avec une valeur de concentration en bêta-amino isobutyrate de référence positive qui est représentative de la concentration en bêta-amino isobutyrate d'un chat présentant une probabilité accrue de développer des calculs d'oxalate de calcium, dans laquelle la concentration en bêta-amino isobutyrate du sujet félin étant égale ou supérieure à la valeur de concentration en bêta-amino isobutyrate de référence positive indique que le sujet félin présente une probabilité accrue de développer des calculs d'oxalate de calcium.

8. Procédé selon l'une quelconque des revendications 1 à 6, comprenant en outre
l'analyse d'un échantillon biologique obtenu à partir du sujet félin pour déterminer la concentration en bêta-amino isobutyrate du sujet félin ;
l'analyse d'un échantillon témoin positif pour déterminer la concentration en bêta-amino isobutyrate de l'échantillon témoin positif, dans laquelle l'échantillon témoin positif contient du bêta-amino isobutyrate à une concentration représentative d'un chat présentant une probabilité accrue de développer des calculs d'oxalate de calcium ; et
la comparaison de la concentration en bêta-amino isobutyrate du sujet félin avec la concentration en bêta-amino isobutyrate de l'échantillon témoin positif, dans laquelle la concentration en bêta-amino isobutyrate du sujet félin étant égale ou supérieure à la concentration en bêta-amino isobutyrate de l'échantillon témoin positif indique que le sujet félin présente une probabilité accrue de développer des calculs d'oxalate de calcium.

9. Procédé selon l'une quelconque des revendications 1 à 8, comprenant en outre :
l'analyse d'un échantillon biologique obtenu à partir du sujet félin pour déterminer la concentration en 2-oxoarginine du sujet félin ; et
la comparaison de la concentration en 2-oxoarginine du sujet félin avec une valeur de concentration en 2-oxoarginine de référence positive qui est représentative de la concentration en 2-oxoarginine d'un chat présentant une probabilité accrue de développer des calculs d'oxalate de calcium, dans laquelle la concentration en 2-oxoarginine du sujet félin étant égale ou inférieure à la valeur de concentration en 2-oxoarginine de référence positive indique que le sujet félin présente une probabilité accrue de développer des calculs d'oxalate de calcium.

10. Procédé selon l'une quelconque des revendications 1 à 8, comprenant en outre :
l'analyse d'un échantillon biologique obtenu à partir du sujet félin pour déterminer la concentration en 2-oxoarginine du sujet félin ;
l'analyse d'un échantillon témoin positif pour déterminer la concentration en 2-oxoarginine de l'échantillon témoin positif, dans laquelle l'échantillon témoin positif contient de la 2-oxoarginine à une concentration représentative d'un chat présentant une probabilité accrue de développer des calculs d'oxalate de calcium ; et
la comparaison de la concentration en 2-oxoarginine du sujet félin avec la concentration en 2-oxoarginine de l'échantillon témoin positif, dans laquelle la concentration en 2-oxoarginine du sujet félin étant égale ou inférieure à la concentration en 2-oxoarginine de l'échantillon témoin positif indique que le sujet félin présente une probabilité accrue de développer des calculs d'oxalate de calcium.

11. Procédé d'identification d'un sujet félin qui présente une probabilité accrue de développer des calculs d'oxalate de calcium, le procédé comprenant :
l'analyse d'un échantillon biologique obtenu à partir du sujet félin pour déterminer la concentration en 2-oxoarginine du sujet félin ; et
la comparaison de la concentration en 2-oxoarginine du sujet félin avec une valeur de concentration en 2-oxoarginine de référence positive qui est représentative de la concentration en 2-oxoarginine d'un chat présentant une probabilité accrue de développer des calculs d'oxalate de calcium, dans laquelle la concentration en 2-oxoarginine du sujet félin étant égale ou inférieure à la valeur de concentration en 2-oxoarginine de référence positive indique que le sujet félin présente une probabilité accrue de développer des calculs d'oxalate de calcium.

12. Procédé d'identification d'un sujet félin qui présente une probabilité accrue de développer des calculs d'oxalate de calcium, le procédé comprenant :
l'analyse d'un échantillon biologique obtenu à partir du sujet félin pour déterminer la concentration en 2-oxoarginine du sujet félin ;
l'analyse d'un échantillon témoin positif pour déterminer la concentration en 2-oxoarginine de l'échantillon témoin positif, dans laquelle l'échantillon témoin positif contient de la 2-oxoarginine à une concentration représentative d'un chat présentant une probabilité accrue de développer des calculs d'oxalate de calcium ; et
la comparaison de la concentration en 2-oxoarginine du sujet félin avec la concentration en 2-oxoarginine de l'échantillon témoin positif, dans laquelle la concentration en 2-oxoarginine du sujet félin étant égale ou inférieure à la concentration en 2-oxoarginine de l'échantillon témoin positif indique que le sujet félin présente une probabilité accrue de développer des calculs d'oxalate de calcium.

13. Composition nutritionnelle à faible teneur en arginine pour une utilisation dans un procédé de réduction du risque de formation de calculs d'oxalate de calcium chez un sujet félin, le procédé comprenant :
l'identification du sujet félin comme étant un sujet félin présentant une probabilité accrue de développer des calculs d'oxalate de calcium selon l'une quelconque des revendications 1 à 12 ; et
l'alimentation du sujet félin avec un régime alimentaire quotidien d'une composition nutritionnelle à faible teneur en arginine, le régime alimentaire quotidien de la composition nutritionnelle à faible teneur en arginine contenant 1,84 % ou moins d'arginine en pourcentage de l'apport quotidien en matière sèche.

14. Composition nutritionnelle à faible teneur en arginine pour une utilisation selon la revendication 13, dans laquelle le régime alimentaire quotidien de la composition nutritionnelle à faible teneur en arginine contient 1,21 % ou moins d'arginine en pourcentage de l'apport quotidien en matière sèche, en particulier 1,11 % ou moins d'arginine en pourcentage de l'apport quotidien en matière sèche, contient plus particulièrement moins de 1,04 % d'arginine en pourcentage de l'apport quotidien en matière sèche.

15. Composition nutritionnelle à faible teneur en arginine pour une utilisation selon la revendication 13, dans laquelle le régime alimentaire quotidien de la composition nutritionnelle à faible teneur en arginine contient 1,04 à 1,84 % d'arginine en pourcentage de l'apport quotidien en matière sèche.
